# EUROPEAN PATENT APPLICATION

(11) **EP 2 168 576 A2**
(43) Date of publication of application: **31.03.2010**
(21) Application number: 09009879.9
(22) Date of filing: 14.09.2001
(51) Int. Cl.: A61K 31/15, A61K 31/155, A61K 31/50, A61K 31/505, A61K 31/44, A61K 31/4402, A61K 31/4965, A61K 31/502, A61K 31/498, A61K 31/47, A61K 31/53, A61P 3/06, A61P 9/10

(54) **New utilities of tricyclic compounds**

(62) Divisional of application: 01967683.2
(71) Applicant: Shionogi & Co., Ltd., Osaka 541-0045 (JP)
(72) Inventor: Ishizuka, Natsuki, Osaka-shi Osaka 553-0002 (JP); Sakai, Katsunori, Toyonaka-shi Osaka 561-0825 (JP); Hayashi, Kunio, Kadoma-shi Osaka 571-0027 (JP)
(74) Representative: Hayes, Adrian Chetwynd

(57) **Abstract**

A pharmaceutical composition for enhancing the expression of apoAI, which comprises a compound of formula (I): 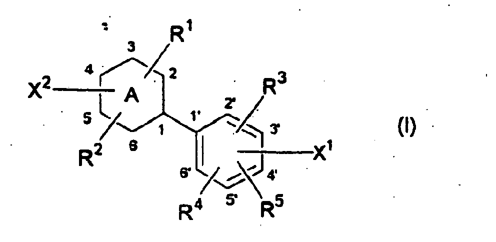 in which.
X¹ and X² are independently an aryl that may be optionally substituted, a heteroaryl that may be optionally substituted, a cycloalkyl that may be optionally substituted, an aryloxy that may be optionally substituted, a heteroaryloxy that may be optionally substituted, an arylthio that may be optionally substituted, a heteroarylthio that may be optionally substituted, a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a carboxy, a lower alkoxycarbonyl that may be optionally substituted, an acyl that may be optionally substituted, an amino that may be optionally substituted, or a carbamoyl that may be optionally substituted, provided that at least one of X¹ and X² is an aryl that may be optionally substituted, or a heteroaryl that may be optionally substituted;
ring A is a pyridine ring, a pyrimidine ring, a pyrazine ring, a pyridazine ring, a triazine ring, a quinoline ring, a quinoxaline ring, or a phthalazine ring;
R¹, R², R³, R⁴ and R⁵ are independently a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a carboxy, a lower alkoxycarbonyl that may be optionally substituted, an acyl that may be optionally substituted, an amino that may be optionally substituted, or a carbamoyl that may be optionally substituted; a prodrug thereof, a pharmaceutically acceptable salt or solvate of them.

## Description

### FILED OF THE INVENTION

This invention relates to a pharmaceutical composition for enhancing the expression of apolipoprotein AI.

### BACKGROUND ART

Cholesterol is well known as a main etiologic factor for arteriosclerosis that causes severe heart diseases. Especially, increased levels of serum low density lipoprotein (LDL) are believed to be a definite risk factor for coronary heart diseases (CHDs). Remedies for decreasing the level of LDL-cholesterol (LDL-C) in plasma by use of statins have been shown to be clinically effective in preventing the onset of CHDs and improving the conditions of CHDs and survivals in patients suffering from hypercholesterolemia. However, about 40 % of CHDs patients have a normal level of LDL-C, and are not always cured effectively by remedies for decreasing the level of LDL-C. On the other hand, it has been known that a half of CHDs patients having a normal level of LDL-C shows a lower level of high density lipoprotein (HDL) cholesterol (HDL-C).

Epidemiological trials in Europe and the U.S. such as Framingham studies and MRFIT (Multiple Risk Factor Intervention Trial) have reported that incidence of coronary heart diseases is higher when the level of HDL-C is lower. Other reports show that patients having only a lower level of HDL-C with normal levels of total cholesterol and triglyceride increased in a risk of arteriosclerosis. Those suggest that a low level of serum HDL-C (less than 35 to 40mg/dl) should be an independent risk factor of CHD, and the risk of complications in coronary artery diseases rapidly increases.

HDL plays an important role in reverse cholesterol transport system that is known as a biological mechanism to transfer an excess cholesterol in cells back to liver so as to maintain the level of cholesterol in living bodies normally.

Lipoproteins such as HDL is mainly comprised of lipids and proteins called apoprotein, and HDL comprises an apoprotein as referred to apolipoprotein AI (hereinafter, made up by apoAI) as a main component.

Excess free cholesterols (FCs) and phospholipids in peripheral cells are extracted by free apoAI to form lipoproteins called preβ-HDL(s). The excess FCs integrated in the preβ-HDLs are transformed into cholesteryl esters (CEs) by lecithin : cholesterol acyl transferase (LCAT), while the preβ-HDLs increase in their particle size to mature into spherical HDLs (HDL3s). The matured HDLs are classified into diverse subfractions based on the density, and these particles further grow up to form HDL2(s). CEs are continuously transferred into very low density lipoprotein (VLDL) and LDL by means of cholesteryl ester-transporter protein (CETP). Those lipoproteins that integrate CEs are finally taken into the liver via receptors. During the course, apoAI is regenerated, and again interacts with peripheral cells to repeat the extracting of cholesterols and the regeneration of preβ-HDLs.

It has been well understood that HDL plays a central role in reverse cholesterol transport system and is a defensive factor of arteriosclerosis. It is expected that agents that promote the HDL functions could be clinically effective as medicaments for treating arteriosclerotic diseases. Accordingly, studies to develop agents that enhance the level of HDL in plasma have been conducted via various approaches.

Among them, one of the most promising approachs is to enhance the serum level of apoAI, a main component of HDL. It is understood that apoAI production increased by enhancing the expression of apoAI gene leads to directly the elevation of HDL-C level in plasma, resulting in the activation of reverse cholesterol transport system. In fact, it has been demonstrated that the mRNA level of apoAI in liver correlates closely with the levels of apoAI and HDL-C in blood (Dueland S., France D., Wang SL., Trawick JD., and Davis RA., J. Lipid Res., 38:1445-53 (1997), "Cholesterol 7alpha-hydroxylase influences the expression of hepatic apoA-I in two inbred mouse strains displaying different susceptibilities to atherosclerosis and in hepatoma cells."). In addition, it has been shown that apoAI-transgenic mice and rabbit pathologic models administered with apoAI exhibit anti-arteriosclerosis activities (Rubin E.M., Krauss R.M., Spangler E. A., Verstuyft J. G., and Clift S. M., Nature 353, 265-267 (1991), "Inhibition of early atherogenesis in transgenic mice by human apolipoprotein AI."; Plump A.S., Scott C.J., Breslow J.L., Proc. Natl. Acad. Sci. USA., 91, 9607-9611 (1994), "Human apolipoprotein A-I gene expression increases high density lipoprotein and suppress atherosclerosis in the apolipoprotein E-deficient mouse. "; Miyazaki A., Sakuma S., Morikawa W., Takiue T., Miake F., Terano T., Sakai M., Hakamata H., Sakamoto Y., et al., Arterioscler. Thromb. Vasc. Biol., 15, 1882-1888 (1995), "Intravenous injection of rabbit apolipoprotein A-I inhibits the progression of atherosclerosis in cholesterol-fed rabbits.").

All those facts clearly suggest that agents that enhance the expression of apoAI would be candidates for medicaments of dyslipidemia, arteriosclerotic diseases, and other diverse diseases involving HDL.

Compounds that elevate HDL are described in WO97/ 19931, WO97/19932, US5599829, EP796874, and Japanese Patent Publication (kokai) No. 255574/1997, whereas compounds that increase apoAI are described in Japanese Patent Publication (kokai) No. 221959/1993, Japanese Patent Publication (kokai) No. 291094/1996, and WO97/09048.

### DISCLOSURE OF THE INVENTION

The present invention is directed to pharmaceutical compositions for enhancing the expression of apoAI used as medicaments for treatment of cardiovascular diseases on the basis of improvement in the functions of HDL.

Specifically, the invention provides
1) A pharmaceutical composition for enhancing the expression of apoAI, which comprises a compound of formula (I): 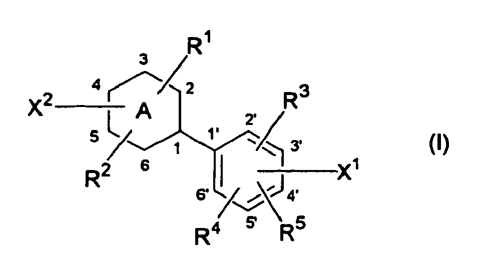 in which
   X¹ and X² are independently an aryl that may be optionally substituted, a heteroaryl that may be optionally substituted, a cycloalkyl that may be optionally substituted, an aryloxy that may be optionally substituted, a heteroaryloxy that may be optionally substituted, an arylthio that may be optionally substituted, a heteroarylthio that may be optionally substituted, a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a carboy, a lower alkoxycarbonyl that may be optionally substituted, an acyl that may be optionally substituted, an amino that may be optionally substituted, or a carbamoyl that may be optionally substituted,
   provided that at least one of X¹ and X² is an aryl that may be optionally substituted, or a heteroaryl that may be optionally substituted;
   ring A is a benzene ring that may be optionally condensed with another aromatic ring, or a 6-membered aromatic heterocyclic ring containing 1 to 3 N atoms that may be optionally condensed with another aromatic ring;
   R¹, R², R³, R⁴ and R⁵ are independently a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a carboxy, a lower alkoxycarbonyl that may be optionally substituted, an acyl that may be optionally substituted, an amino that may be optionally substituted, or a carbamoyl that may be optionally substituted (herein after as referred to compound (I)); a prodrug thereof, a pharmaceutically acceptable salt or solvate of them;
2) The pharmaceutical composition according to above 1), in which ring A in formula (I) is a benzene ring, a pyridine ring, a pyrimidine ring, a pyrazine ring, a pyridazine ring, a triazine ring, a quinoline ring, a quinoxaline ring, or a phthalazine ring; X¹ is a phenyl that may be optionally substituted, or a heteroaryl that may be optionally substituted; and X² is a hydrogen, a halogen, a hydroxy, a lower alkyl, a lower alkenyl, a lower alkoxy, or a phenyl;
3) The pharmaceutical composition according to above 1) or 2), in which X¹ in formula (I) is bonded at position 4';
4) The pharmaceutical composition according to any one of above 1) to 3), in which X¹ in formula (I) is a phenyl that may be optionally substituted, a furyl that may be optionally substituted, or a thienyl that may be optionally substituted;
5) The pharmaceutical composition according to any one of above 1) to 4), in which at least one of the atoms within ring A in formula (I) at positions 2 and 6 is N;
6) The pharmaceutical composition according to any one of above 1) to 5), in which R¹, R², R³, R⁴ and R⁵ are independently a hydrogen, a hydroxy, a lower alkyl, or a lower alkoxy;
7) The pharmaceutical composition according to above 1), in which ring A in formula (I) is a benzene ring, a pyridine ring, a pyrimidine ring, a pyrazine ring, a pyridazine ring, or a triazine ring; X¹ is a hydrogen, a halogen, a hydroxy, a lower alkyl, a lower alkenyl, a lower alkoxy, or a phenyl; and X² is a phenyl that may be optionally substituted, or a heteroaryl that may be optionally substituted;
8) The pharmaceutical composition according to any one of above 1) to 7), in which X² in formula (I) is bonded at position 4 on ring A;
9) The pharmaceutical composition according to any one of above 1), 7) and 8, in which X² in formula (I) is a phenyl that may be optionally substituted;
10) The pharmaceutical composition according to any one of claims 7 to 9, in which all of R¹, R², R³, R⁴ and R⁵ are a hydrogen;
11) The pharmaceutical composition according to any one of above 1) to 10), which is used for prevention and/or treatment of dyslipidemia or arteriosclerotic diseases;
12) A method of enhancing the expression of apoAI, which comprises administrating a therapeutically effective amount of a compound of formula (I) as defined in above (1), a prodrug thereof, a pharmaceutically acceptable salt or solvate of them to a patient expected to enhance the expression of apoAI; preferably, the method which comprises administrating a therapeutically effective amount of a compound of formula (I) as defined in above (2) to (10), a prodrug thereof, a pharmaceutically acceptable salt or solvate of them;
13) A method of treatment and/or prevention of dyslipidemia, arteriosclerotic diseases or coronary artery diseases, which comprises administrating a therapeutically effective amount of a compound of formula (I) as defined in above (1), a prodrug thereof, a pharmaceutically acceptable salt or solvate of them to a patient suspected to have dyslipidemia, arteriosclerotic diseases or coronary artery diseases; preferably, the method which comprises administrating a therapeutically effective amount of a compound of formula (I) as defined in above (2) to (10), a prodrug thereof, a pharmaceutically acceptable salt or solvate of them;
14) Use of a compound of formula (I) as defined in above (1), a prodrug thereof, a pharmaceutically acceptable salt or solvate of them for the manufacturing a medicament of enhancing the expression of apoAI; preferably, the use of a compound of formula (I) as defined in above (2) to (10), a prodrug thereof, a pharmaceutically acceptable salt or solvate of them;
15) Use of a compound of formula (I) as defined in above (1), a prodrug thereof, a pharmaceutically acceptable salt or solvate of them for the manufacturing a medicament of treatment and/or prevention of dyslipidemia, arteriosclerotic diseases or coronary artery diseases; preferably, the use of a compound of formula (I) as defined in above (2) to (10), a prodrug thereof, a pharmaceutically acceptable salt or solvate of them.

The term "halogen" as used herein includes fluorine, chlorine, bromine and iodine.

The term "lower alkyl" as used herein refers to a straight or branched chain alkyl comprising 1 to 6 carbon atoms, preferably 1 to 3 carbon atoms. Examples of the lower alkyl include methyl, ethyl, n-propyl, isopropyl, n-butyl, isobutyl, sec-butyl, tert-butyl, n-pentyl, isopentyl, neopentyl, hexyl, isohexyl, and the like.

The term "lower alkyl that may be optionally substituted" as used herein includes a lower alkyl, of which any position may be substituted by one or more substituents. The substituent includes a halogen, a hydroxy, a lower alkoxy, an acyl, an acyloxy, a carboxy, a lower alkoxycarbonyl, an amino, a lower alkylamino, an imino that may be optionally substituted wherein the substituent is a heterocyclic ring that may be optionally substituted by hydroxy or a lower alkyl; a nitro, an aryl, a heteroaryl, and the like.

Alkyl moiety of "lower alkoxy", "lower alkylthio" or "lower alkylamino" is similar to the "lower alkyl" as described above.

Substituent in "lower alkoxy that may be optionally substituted" is similar to the substituent of "lower alkyl that may be optionally substituted" as described above.

The term "lower alkylenedioxy" specifically includes methylenedioxy and ethylenedioxy.

Substituent in "lower alkylenedioxy that may be optionally substituted" includes the substituent of "lower alkyl" as described above, and a lower alkenyl and a lower alkynyl.

Lower alkyl moiety of "lower alkoxycarbonyl" is similar to the "lower alkyl" as described above, and substituent of "lower alkoxycarbonyl that may be optionally substituted" is similar to the substituent of "lower alkyl that may be optionally substituted" as described above.

The term "lower alkenyl" refers to a straight or branched alkenyl comprising 2 to 6 carbon atoms, preferably 2 to 4 carbon atoms. Specific examples include vinyl, 1-propenyl, allyl, isopropenyl, butenyl, isobutenyl, butadienyl, pentenyl, isopentenyl, pentadienyl, hexenyl, and isohexenyl, all of which contain one or more double bonds at arbitrary position.

Substituent of "lower alkenyl that may be optionally substituted" includes a hydroxy, a halogen, a lower alkoxy, a carboxy, an acyl, an acyloxy, a cycloalkyl, a lower alkoxycarbonyl, an aryl, and a heteroaryl, and may be substituted at one or more arbitrary positions.

Lower alkenyl moiety of "lower alkenyloxy" and "lower alkenyloxycarbonyl", and substituent of "lower alkenyloxy that may be optionally substituted" are similar to those of "lower alkenyl" and "lower alkenyl that may be optionally substituted" as described above.

The term "lower alkynyl" refers to a straight or branched alkynyl comprising 2 to 6 carbon atoms, preferably 2 to 4 carbon atoms, and specific examples include ethynyl, propynyl, butynyl, pentynyl, hexynyl, heptynyl, octynyl, nonynyl, and decynyl. Those contain one or more triple bonds, and further may contain a double bond.

Substituent of "lower alkynyl that may be optionally substituted" are similar to the substituent of "lower alkyl that may be optionally substituted" as described above.

Lower alkynyl moiety and substituent of "lower alkynyloxy" and "lower alkynyloxy that may be optionally substituted" are similar to those of "lower alkynyl" and "lower alkynyl that may be optionally substituted" as described above, respectively.

The term "acyl" includes an aroyl and an aliphatic acyl containing 1 to 7 carbon atoms. Here, "aroyl" refers to a group wherein a carbonyl group is bonded to an aryl or a heteroaryl group. Examples of the acyl include formyl, acetyl, propionyl, butyryl, isobutyryl, valery, pivaloyl, hexanoyl, acryloyl, propiolyl, methacryloyl, crotonoyl, and benzoyl. Preferably, acetyl and benzoyl are exemplified.

Substituent of "acyl that may be optionally substituted" is similar to the substituent of the "lower alkyl that may be optionally substituted" as described above. The aromatic moiety of aroyl may be substituted by a lower alkyl. Acyl may be substituted at one or more positions by such a substituent.

Acyl moiety of "acyloxy" and "halogenoacyl" is similar to the "acyl" as described above.

The term "amino that may be optionally substituted" includes an amino that may be optionally substituted by one to three substituent(s). Examples of the substituents include the substituents of the "lower alkyl that may be optionally substituted" as described above, and a lower alkyl. Amino substituted by three substituents refers to a quaternary salt. Preferably, an unsubstituted amino, and an amino that is substituted by one or two alkyl(s) and/or acyl(s).

Substituent of "carbamonyl that may be optionally substituted" is similar to the substituent of the "lower alkyl that may be optionally substituted" as described above. Preferably, an unsubstituted carbamoyl and a di-lower alkylcarbamoyl are exemplified.

The term "cycloalkyl" refers to an aliphatic cyclic carbon ring group containing 3 to 10 carbon atoms, preferably 3 to 6 carbon atoms. Examples includes cyclopropyl, cyclobutyl, cyclopentyl, cyclohexyl, cycloheptyl, cyclooctyl, cyclononyl, and cyclodecyl.

Substituent of "cycloalkyl that may be optionally substituted" is similar to the substituent of the "lower alkyl that may be optionally substituted" as described above.

The term "aryl" includes, for example, phenyl, naphthyl, and anthryl. Aryl also includes a phenyl condensed with a non-aromatic carbon ring such as indanyl and indenyl. Phenyl and naphthyl are preferable, and phenyl is most preferable.

The term "heteroaryl" refers to a monocyclic and bicyclic aromatic heterocyclic ring group containing one or more hetero atoms selected from the group consisting of N, S and O within its ring. Examples of the heteroaryl include a monocyclic heteroaryl, e.g., pyrrolyl, imidazolyl, pyrazolyl, pyridyl, pyridazinyl, pyrimidyl, pyrazinyl, triazolyl, triazinyl, tetrazolyl, isoxazolyl, oxazolyl, oxadiazolyl, isothiazolyl, thiazolyl, thiadiazolyl, furyl, and thienyl; as well as a bicyclic heteroaryl, e.g., indolyl, isoindolyl, indolizinyl, benzimidazolyl, indazolyl, cinnolinyl, phthalazinyl, benzoxazolyl, benzisoxazolyl, benzoxadiazolyl, benzothiazolyl, benzisothiazolyl, benzothiadiazolyl, benzofuryl, isobenzofuryl, benzothienyl, benzotriazolyl, imidazopyridyl, triazolopyridyl, imidazothiazolyl, pyrazinopiridazinyl, quinazolinyl, quinolyl, isoquinolyl, quinoxalinyl, purinyl, pteridinyl, naphthylidinyl, pyrazinopyridazinyl, and the like.

Examples of substituents of "aryl that may be optionally substituted", "phenyl that may be optionally substituted", "heteroaryl that may be optionally substituted", "furyl that may be optionally substituted" and "thienyl that may be optionally substituted" include a halogen; a hydroxy; a lower alkyl optionally substituted by a halogen, a hydroxy or a lower alkoxy; a lower alkoxy optionally substituted by a substituent such as a halogen, a hydroxy, an amino, an imino, a hydroxyimino, phenyl, or a heterocyclic imino that may be substituted by a lower alkyl; a lower alkenyl optionally substituted by a halogen, a hydroxy, a lower alkoxy; a lower alkenyloxy optionally substituted by a halogen, a hydroxy or a lower alkoxy; a lower alkynyl optionally substituted by a halogen, a hydroxy or a lower alkoxy; a lower alkynyloxy optionally substituted by a halogen, a hydroxy or a lower alkoxy; a cycloalkyl optionally substituted by a halogen, a hydroxy, a lower alkyl or a lower alkoxy; an acyl; an acyloxy; a carboxy; a lower alkoxycarbonyl; a lower alkenyloxycarbonyl; an amino optionally substituted by a lower alkyl, an acyl or halogenoacyl; a hydrazino; a carbamoyl optionally substituted by a lower alkyl; a nitro; a cyano; a lower alkenyloxy optionally substituted by a halogen, a hydroxy or a lower alkoxy; a mercapto; a lower alkylthio; an aryl optionally substituted by a halogen, a hydroxy, a lower alkyl or a lower alkoxy; a heteroaryl optionally substituted by a halogen, a hydroxy, a lower alkyl or a lower alkoxy; an aryloxy optionally substituted by a halogen, a hydroxy, a lower alkyl or a lower alkoxy; a heteroaryloxy optionally substituted by a halogen, a hydroxy, a lower alkyl or a lower alkoxy; a phenylamino optionally substituted by a halogen, a hydroxy, a lower alkyl or a lower alkoxy; and a lower alkylenedioxy optionally substituted by a halogen, a hydroxy, a lower alkyl, a lower alkenyl, a lower alkynyl, a lower alkoxy, and the like, which may be substituted at one or more arbitrary position. Preferably, a halogen; a hydroxy; a lower alkyl; a lower alkoxy; an acyl; an acyloxy; an amino; an acylamino; a phenyl optionally substituted by a lower alkyl or a lower alkoxy; or a heteroaryl are exemplified.

Aryl moiety of "aryloxy" and "arylthio" is similar to "aryl" as described above.

Substituent of "aryloxy that may be optionally substituted" and "arylthio that may be optionally substituted" are similar to the substituent of "aryl that may be optionally substituted" as described above.

Heteroaryl moiety of "heteroaryloxy" and "heteroarylthio" is similar to "heteroaryl" as described above, and substituent of "heteroaryloxy that may be optionally substituted" and "heteroarylthio that may be optionally substituted" are similar to those of "heteroaryl that may be optionally substituted" as described above.

Specific examples of "6-membered aromatic heterocyclic ring containing 1 to 3 N atoms" include a pyridine ring, a pyrimidine ring, a pyridazine ring, a pyrazine ring, and a triazine ring.

The terms "benzene ring that may be optionally condensed with another aromatic ring" and "6-membered aromatic heterocyclic ring containing 1 to 3 N atoms that may be optionally condensed with another aromatic ring" include a benzene ring and 6-membered aromatic heterocyclic ring containing 1 to 3 N atoms, both of which may be optionally condensed with a benzene ring or monocyclic aromatic heterocyclic ring.

The term "monocyclic aromatic heterocyclic ring" includes a monocyclic aromatic heterocyclic ring group containing one or more hetero atoms selected from the group consisting of N, S and O within its ring.

Examples include pyrrole, imidazole, pyrazole, pyridine, pyridazine, pyrimidine, pyrazine, triazole, triazine, tetrazole, isoxazole, oxazole, oxadiazole, isothiazole, thiazole, thiadiazole, furan, and thiophene.

Examples of "benzene ring that may be optionally condensed with another aromatic ring" include naphthalene, benzofuran, benzimidazole, benzothiazole, benzisothiazole, benzoxazole, benzisoxazole, benzotriazole, benzoxadiazole, benzopyrazole, benzothiophene, benzothiazole, cinnoline, indazole, indole, indoline, isobenzofurane, isoindole, isoindoline, quinoline, isoquinoline, quinoxaline, quinazoline, and phthalazine. Preferably, quinoline, quinoxaline, and phthalazine are exemplified.

Examples of "6-membered aromatic heterocyclic ring containing 1 to 3 N atoms that may be optionally condensed with another aromatic ring" include quinoline, isoquinoline, quinoxaline, quinazoline, phthalazine, cinnoline, naphthyridine, pteridine, purine, and pyrazinopyridazine. Preferably; quinoline, quinoxaline, and phthalazine are exemplified.

The phrase "at least one of the atoms within ring A at positions 2 and 6 is N" means specifically ring A of the followings:

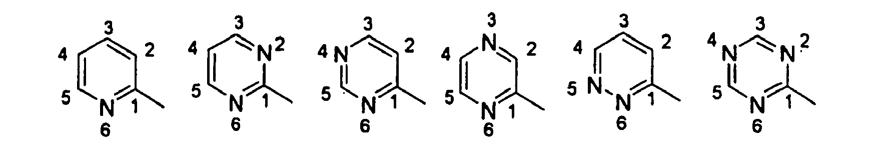

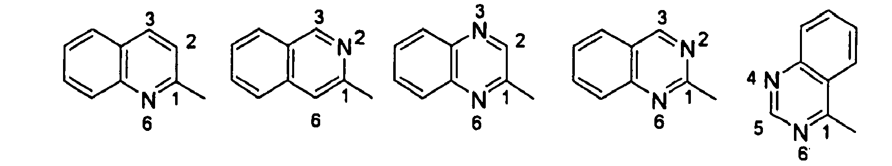

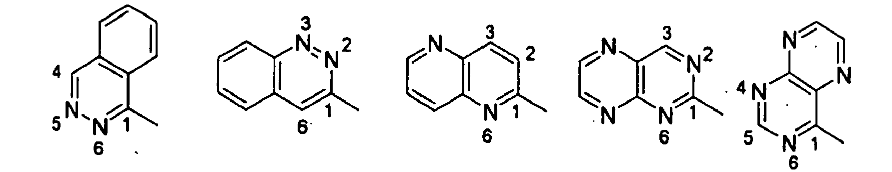

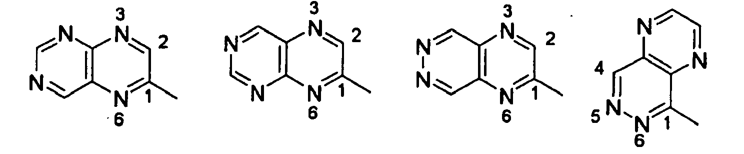

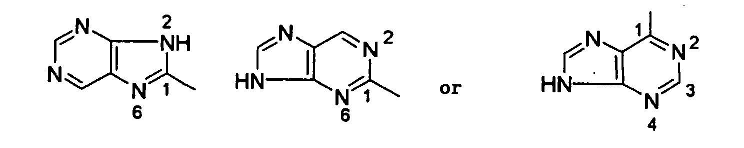

The term "heterocyclic ring" includes the "heteroaryl" as described above, and a non-aromatic heterocyclic ring group.

The term "non-aromatic heterocyclic ring group" include a monocyclic and bicyclic non-aromatic heterocyclic ring group containing one or more hetero atoms selected from the group consisting of N, S and O within its ring. Examples of the non-aromatic heterocyclic ring include oxanyl, thiiranyl, oxiranyl, oxathiolanyl, azetidinyl, thianyl, pyrrolidinyl, pyrrolinyl, imidazolidinyl, imidazolinyl, pyrazolidinyl, pyrazolinyl, piperidyl, piperazinyl, and morpholinyl.

The compounds according to the invention include pharmaceutically acceptable, producible salts. Examples of the "pharmaceutically acceptable salts" include a salt with an inorganic acid e.g. those with hydrochloric acid, sulfuric acid, nitric acid, phosphoric acid, or the like; a salt with an organic acid e.g. those with p-toluenesulfonic acid, methanesulfonic acid, oxalic acid, citric acid, or the like; a salt with an organic base e.g. ammonium, trimethylammonium, triethylammonium, or the like; a salt with an alkaline metal e.g. sodium or potassium, or the like; a quaternary salt with alkyl halide e.g., methyl iodide, ethyl iodide or the like; and a salt with an alkaline earth metal e.g., calcium or magnesium, or the like.

The compounds according to the invention may form solvates as coordinated with a suitable organic solvent and/or water. Hydrates are exemplified.

The compounds according to the invention also include prodrugs. In the context of the invention, a "prodrug" is a derivative of a compound according to the invention comprising a chemically or metabolically cleavable group. In the course of metabolism in the body, a prodrug shows a pharmacological activity as a result of conversion to the compounds according to the invention. Method for selecting and producing suitable prodrug derivatives are described in, e.g. "Design of Prodrugs, Elsevier, Amsterdam (1985)".

Prodrugs of a compound according to the invention having a carboxy are exemplified by an ester derivative prepared by condensing the carboxy group with a suitable alcohol, e.g., COOR^{A} wherein R^{A} is a lower alkyl, a lower alkenyl or an aryl, each of which may be optionally substituted in which the substituent may be a hydroxy, an acyloxy, a carboxy, a sulfonic acid, an amino, a lower alkylamino, or the like; or alternatively by an amide derivative prepared by reacting the carboxy and a suitable amine, e.g., CONR^{B}R^{C} wherein R^{B} is a hydrogen, a lower alkyl, or the like; and R^{C} is a hydrogen, a lower alkyl, an amino, a hydroxy, or the like.

Prodrugs of a compound according to the invention having a hydroxy are exemplified by an acyloxy derivative prepared by reacting the hydroxy group and a suitable acyl halide or a suitable acid anhydride, e.g., -OCOR^{A} wherein R^{A} is as defined above.

Prodrugs of a compound according to the invention having an amino, are exemplified by an amide derivative prepared by reacting the amino group and a suitable acid halide or a suitable mixed anhydride compound, e.g., NHCOR^{A}, and NHCOOR^{A} wherein R^{A} is as defined above.

When compound (I) according to the invention has asymmetric carbon atom(s), then the invention encompasses a racemic mixture, both of enantiomers, and all of diastereomers. When compound (I) according to the invention has a double bond, the invention may include both of geometric isomers resulting from possible arrangements of its substituents.

Although all of the compounds according to the invention have an activity for enhancing the expression of apoAI, the following compounds can be listed as preferable compounds.
(a) in case that X¹ is an aryl that may be optionally substituted or a heteroaryl that may be optionally substituted:
   a-1) a compound wherein X¹ is an aryl that may be optionally substituted or a heteroaryl that may be optionally substituted, which is bonded at position 4' (hereinafter, X¹ is regarded as X1-1);
      a compound wherein X¹ is a phenyl that may be optionally substituted or a monocyclic heteroaryl that may be optionally substituted, which is bonded at position 4' (hereinafter, X¹ is regarded as X1-2);
      a compound wherein X¹ is a phenyl that may be optionally substituted, a furyl that may be optionally substituted, or a thienyl that may be optionally substituted, which is bonded at position 4' (hereinafter, X¹ is regarded as X1-3);
      a compound wherein X¹ is a phenyl that may be optionally substituted wherein the substituent is a halogen; a hydroxy; a lower alkyl; a lower alkoxy that may be optionally substituted by an amino, a hydroxyimino, a lower alkylpiperazinylimino, or a phenyl; a lower alkylenedioxy that may be optionally substituted by a halogen, a hydroxy, a lower alkyl, a lower alkenyl, a lower alkynyl or a lower alkoxy; an acyloxy, and an acylamino; an unsubstituted furyl; or an unsubstituted thienyl, which is bonded at position 4' (hereinafter, X¹ is regarded as X1-4);
      a compound wherein X¹ is a phenyl that may be optionally substituted wherein the substituent is a halogen, a lower alkyl, a lower alkoxy, a lower alkylenedioxy, an acyloxy, and an acylamino, which is bonded at position 4' (hereinafter, X¹ is regarded as X1-5);
   a-2) a compound wherein X² is a phenyl, a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a carboxy, a lower alkoxycarbonyl that may be optionally substituted, an acyl that may be optionally substituted, an amino that may be optionally substituted, or a carbamoyl that may be optionally substituted (hereinafter, X² is regarded as X2-1);
      a compound wherein X² is a phenyl, a hydrogen, a halogen, a hydroxy, a lower alkyl, a halogeno-lower alkyl, a hydroxy-lower alkyl, a lower alkoxy-lower alkyl, an acyl-lower alkyl, a lower alkenyl, a lower alkoxy, a halogeno-lower alkoxy, a hydroxy-lower alkoxy, a lower alkoxy-lower alkoxy, an acyl-lower alkoxy, a carboxy, a lower alkoxycarbonyl, an acyl, an amino, a lower alkylamino or an acylamino (hereinafter, X² is regarded as X2-2);
      a compound wherein X² is a hydrogen, a halogen, a hydroxy, a lower alkyl, a lower alkenyl, or a lower alkoxy (hereinafter, X² is regarded as X2-3);
      a compound wherein X² is a hydrogen, a halogen, a lower alkyl, or a lower alkoxy (hereinafter, X² is regarded as X2-4);
   a-3) a compound wherein ring A is a benzene ring that may be optionally condensed with another aromatic ring, or a 6-membered aromatic heterocyclic ring containing 1 to 3 N atoms that may be optionally condensed with another aromatic ring (hereinafter, ring A is regarded as A-1);
      a compound wherein ring A is a benzene ring, or a 6-membered aromatic heterocyclic ring containing 1 to 3 N atoms wherein at least one of the atoms at positions 2 and 6 is N, which may be optionally condensed with a benzene ring (hereinafter, ring A is regarded as A-2);
      a compound wherein ring A is a benzene ring, a pyridine ring, a pyrimidine ring, a pyrazine ring, a pyridazine ring, a triazine ring, a quinoline ring, a quinoxaline ring, or a phthalazine ring (hereinafter, ring A is regarded as A-3);
      a compound wherein ring A is 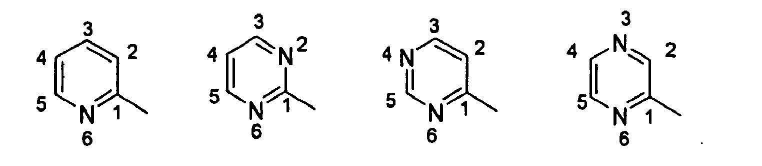 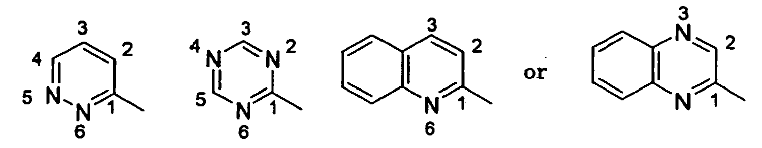 (hereinafter, ring A is regarded as A-4);
   a-4) a compound wherein R¹, R², R³, R⁴ and R⁵ are independently a hydrogen, a halogen, a hydroxy, a lower alkyl that may be optionally substituted, a lower alkenyl, a lower alkoxy, a carboxy, a lower alkoxycarbonyl, an acyl, an amino, a lower alkylamino, or an acylamino (hereinafter, R¹ to R⁵ are regarded as R-1);
      a compound wherein R¹, R², R³, R⁴ and R⁵ are independently a hydrogen, a halogen, a hydroxy, a lower alkyl, a lower alkoxy, an acyl, an amino, a lower alkylamino, or an acylamino (hereinafter, R¹ to R⁵ are regarded as R-2);
      a compound wherein R¹, R², R³, R⁴ and R⁵ are independently a hydrogen, a halogen, a lower alkyl, or a lower alkoxy (hereinafter, R¹ to R⁵ are regarded as R-3);
      a compound wherein R¹, R², R³, R⁴ and R⁵ are all a hydrogen (hereinafter, R¹ to R⁵ are regarded as R-4);
(b) in case that X² is an aryl that may be optionally substituted or a heteroaryl that may be optionally substituted:
   b-1) a compound wherein X¹ is a phenyl, a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a lower alkynyloxy that may be optionally substituted, a carboxy, a lower alkoxycarbonyl that may be optionally substituted, an acyl that may be optionally substituted, an amino that may be optionally substituted, or a carbamoyl that may be optionally substituted (hereinafter, X¹ is regarded as X1-6);
      a compound wherein X¹ is a phenyl, a hydrogen, a halogen, a hydroxy, a lower alkyl, a halogeno-lower alkyl, a hydroxy-lower alkyl, a lower alkoxy-lower alkyl, an acyl-lower alkyl, a lower alkenyl, a lower alkoxy, a halogeno-lower alkoxy, a hydroxy-lower alkoxy, a lower alkoxy-lower alkoxy, an acyl-lower alkoxy, a lower alkenyloxy, a carboxy, a lower alkoxycarbonyl, an acyl, an amino, a lower alkylamino or an acylamino (hereinafter, X¹ is regarded as X 1-7);
      a compound wherein X¹ is a hydrogen, a halogen, a hydroxy, a lower alkyl, a lower alkenyl, or a lower alkoxy (hereinafter, X¹ is regarded as X1-8);
      a compound wherein X¹ is a hydrogen (hereinafter, X¹ is regarded as X1-9);
   b-2) a compound wherein X² is an aryl that may be optionally substituted or a heteroaryl that may be optionally substituted, which is bonded at position 4' (hereinafter, X² is regarded as X2-5);
      a compound wherein X² is a phenyl that may be optionally substituted or a monocyclic heteroaryl that may be optionally substituted, which is bonded at position 4' (hereinafter, X² is regarded as X2-6);
      a compound wherein X¹ is a phenyl that may be optionally substituted, a furyl that may be optionally substituted, or a thienyl that may be optionally substituted, which is bonded at position 4' (hereinafter, X² is regarded as X2-7);
      a compound wherein X² is a phenyl that may be optionally substituted wherein the substituent is a hydroxy, a lower alkyl, a lower alkoxy, an acyloxy, a lower alkylamino, or an acylamino; an unsubstituted furyl; or an unsubstituted thienyl, which is bonded at position 4' (hereinafter, X² is regarded as X2-8);
      a compound wherein X² is an unsubstituted phenyl which is bonded at position 4' (hereinafter, X² is regarded as X2-9);
   b-3) a compound wherein ring A is a benzene ring or a 6-membered aromatic heterocyclic ring containing 1 to 3 N atoms (hereinafter, ring A is regarded as A-5);
      a compound wherein ring A is a 6-membered aromatic heterocyclic ring containing 1 to 3 N atoms (hereinafter, ring A is regarded as A-6);
      a compound wherein ring A is a benzene ring, a pyridine ring, a pyrimidine ring, a pyrazine ring, a pyridazine ring, or a triazine ring (hereinafter, ring A is regarded as A-7);
   b-4) a compound wherein R¹, R², R³, R⁴ and R⁵ are R-1;
      a compound wherein R¹, R², R³, R⁴ and R⁵ are R-2;
      a compound wherein R¹, R², R³, R⁴ and R⁵ are R-3;
      a compound wherein R¹, R², R³, R⁴ and R⁵ are R-4;
      a compound wherein a combination of X¹, X², A and R, (X¹, X², A, R) is as follows:
      (X¹, X², A, R) = (X1-1, X2-1, A-1, R-1), (X1-1, X2-1, A-1, R-2), (X1-1, X2-1, A-1, R-3), (X1-1, X2-1, A-1, R-4), (X1-1, X2-1, A-2, R-1), (X1-1, X2-1,
      A-2, R-2), (X1-1, X2-1, A-2, R-3), (X1-1, X2-1, A-2, R-4), (X1-1, X2-1, A-3, R-1), (X1-1, X2-1, A-3, R-2), (X1-1, X2-1, A-3, R-3), (X1-1, X2-1, A-3,
      R-4), (X1-1, X2-1, A-4, R-1), (X1-1, X2-1, A-4, R-2), (X1-1, X2-1, A-4, R-3), (X1-1, X2-1, A-4, R-4), (X1-1, X2-2, A-1, R-1), (X1-1, X2-2, A-1, R-2),
      (X1-1, X2-2, A-1, R-3), (X1-1, X2-2, A-1, R-4), (X1-1, X2-2, A-2, R-1),
      (X1-1, X2-2, A-2, R-2), (X1-1, X2-2, A-2, R-3), (X1-1, X2-2, A-2, R-4),
      (X1-1, X2-2, A-3, R-1), (X1-1, X2-2, A-3, R-2), (X1-1, X2-2, A-3, R-3),
      (X1-1, X2-2, A-3, R-4), (X1-1, X2-2, A-4, R-1), (X1-1, X2-2, A-4, R-2),
      (X1-1, X2-2, A-4, R-3), (X1-1, X2-2, A-4, R-4), (X1-1, X2-3, A-1, R-1),
      (X1-1, X2-3, A-1, R-2), (X1-1, X2-3, A-1, R-3), (X1-1, X2-3, A-1, R-4),
      (X1-1, X2-3, A-2, R-1), (X1-1, X2-3, A-2, R-2), (X1-1, X2-3, A-2, R-3),
      (X1-1, X2-3, A-2, R-4), (X1-1, X2-3, A-3, R-1), (X1-1, X2-3, A-3, R-2),
      (X1-1, X2-3, A-3, R-3), (X1-1, X2-3, A-3, R-4), (X1-1, X2-3, A-4, R-1),
      (X1-1, X2-3, A-4, R-2), (X1-1, X2-3, A-4, R-3), (X1-1, X2-3, A-4, R-4),
      (X1-1, X2-4, A-1, R-1), (X1-1, X2-4, A-1, R-2), (X1-1, X2-4, A-1, R-3),
      (X1-1, X2-4, A-1, R-4), (X1-1, X2-4, A-2, R-1), (X1-1, X2-4, A-2, R-2),
      (X1-1, X2-4, A-2, R-3), (X1-1, X2-4, A-2, R-4), (X1-1, X2-4, A-3, R-1),
      (X1-1, X2-4, A-3, R-2), (X1-1, X2-4, A-3, R-3), (X1-1, X2-4, A-3, R-4),
      (X1-1, X2-4, A-4, R-1), (X1-1, X2-4, A-4, R-2), (X1-1, X2-4, A-4, R-3),
      (X1-1, X2-4, A-4, R-4),
      (X1-2, X2-1, A-1, R-1), (X1-2, X2-1, A-1, R-2), (X1-2, X2-1, A-1,
      R-3), (X1-2, X2-1, A-1, R-4), (X1-2, X2-1, A-2, R-1), (X1-2, X2-1, A-2, R-2), (X1-2, X2-1, A-2, R-3), (X1-2, X2-1, A-2, R-4), (X1-2, X2-1, A-3, R-1),
      (X1-2, X2-1, A-3, R-2), (X1-2, X2-1, A-3, R-3), (X1-2, X2-1, A-3, R-4),
      (X1-2, X2-1, A-4, R-1), (X1-2, X2-1, A-4, R-2), (X1-2, X2-1, A-4, R-3),
      (X1-2, X2-1, A-4, R-4), (X1-2, X2-2, A-1, R-1), (X1-2, X2-2, A-1, R-2),
      (X1-2, X2-2, A-1, R-3), (X1-2, X2-2, A-1, R-4), (X1-2, X2-2, A-2, R-1),
      (X1-2, X2-2, A-2, R-2), (X1-2, X2-2, A-2, R-3), (X1-2, X2-2, A-2, R-4),
      (X1-2, X2-2, A-3, R-1), (X1-2, X2-2, A-3, R-2), (X1-2, X2-2, A-3, R-3),
      (X1-2, X2-2, A-3, R-4), (X1-2, X2-2, A-4, R-1), (X1-2, X2-2, A-4, R-2),
      (X1-2, X2-2, A-4, R-3), (X1-2, X2-2, A-4, R-4), (X1-2, X2-3, A-1, R-1),
      (X1-2, X2-3, A-1, R-2), (X1-2, X2-3, A-1, R-3), (X1-2, X2-3, A-1, R-4),
      (X1-2, X2-3, A-2, R-1), (X1-2, X2-3, A-2, R-2), (X1-2, X2-3, A-2, R-3),
      (X1-2, X2-3, A-2, R-4), (X1-2, X2-3, A-3, R-1), (X1-2, X2-3, A-3, R-2),
      (X1-2, X2-3, A-3, R-3), (X1-2, X2-3, A-3, R-4), (X1-2, X2-3, A-4, R-1),
      (X1-2, X2-3, A-4, R-2), (X1-2, X2-3, A-4, R-3), (X1-2, X2-3, A-4, R-4),
      (X1-2, X2-4, A-1, R-1), (X1-2, X2-4, A-1, R-2), (X1-2, X2-4, A-1, R-3),
      (X1-2, X2-4, A-1, R-4), (X1-2, X2-4, A-2, R-1), (X1-2, X2-4, A-2, R-2),
      (X1-2, X2-4, A-2, R-3), (X1-2, X2-4, A-2, R-4), (X1-2, X2-4, A-3, R-1),
      (X1-2, X2-4, A-3, R-2), (X1-2, X2-4, A-3, R-3), (X1-2, X2-4, A-3, R-4),
      (X1-2, X2-4, A-4, R-1), (X1-2, X2-4, A-4, R-2), (X1-2, X2-4, A-4, R-3),
      (X1-2, X2-4, A-4, R-4),
      (X1-3, X2-1, A-1, R-1), (X1-3, X2-1, A-1, R-2), (X1-3, X2-1, A-1,
      R-3), (X1-3, X2-1, A-1, R-4), (X1-3, X2-1, A-2, R-1), (X1-3, X2-1, A-2, R-2), (X1-3, X2-1, A-2, R-3), (X1-3, X2-1, A-2, R-4), (X1-3, X2-1, A-3, R-1),
      (X1-3, X2-1, A-3, R-2), (X1-3, X2-1, A-3, R-3), (X1-3, X2-1, A-3, R-4),
      (X1-3, X2-1, A-4, R-1), (X1-3, X2-1, A-4, R-2), (X1-3, X2-1, A-4, R-3),
      (X1-3, X2-1, A-4, R-4), (X1-3, X2-2, A-1, R-1), (X1-3, X2-2, A-1, R-2),
      (X1-3, X2-2, A-1, R-3), (X1-3, X2-2, A-1, R-4), (X1-3, X2-2, A-2, R-1),
      (X1-3, X2-2, A-2, R-2), (X1-3, X2-2, A-2, R-3), (X1-3, X2-2, A-2, R-4),
      (X1-3, X2-2, A-3, R-1), (X1-3, X2-2, A-3, R-2), (X1-3, X2-2, A-3, R-3),
      (X1-3, X2-2, A-3, R-4), (X1-3, X2-2, A-4, R-1), (X1-3, X2-2, A-4, R-2),
      (X1-3, X2-2, A-4, R-3), (X1-3, X2-2, A-4, R-4), (X1-3, X2-3, A-1, R-1),
      (X1-3, X2-3, A-1, R-2), (X1-3, X2-3, A-1, R-3), (X1-3, X2-3, A-1, R-4),
      (X1-3, X2-3, A-2, R-1), (X1-3, X2-3, A-2, R-2), (X1-3, X2-3, A-2, R-3),
      (X1-3, X2-3, A-2, R-4), (X1-3, X2-3, A-3, R-1), (X1-3, X2-3, A-3, R-2),
      (X1-3, X2-3, A-3, R-3), (X1-3, X2-3, A-3, R-4), (X1-3, X2-3, A-4, R-1),
      (X1-3, X2-3, A-4, R-2), (X1-3, X2-3, A-4, R-3), (X1-3, X2-3, A-4, R-4),
      (X1-3, X2-4, A-1, R-1), (X1-3, X2-4, A-1, R-2), (X1-3, X2-4, A-1, R-3),
      (X1-3, X2-4, A-1, R-4), (X1-3, X2-4, A-2, R-1), (X1-3, X2-4, A-2, R-2),
      (X1-3, X2-4, A-2, R-3), (X1-3, X2-4, A-2, R-4), (X1-3, X2-4, A-3, R-1),
      (X1-3, X2-4, A-3, R-2), (X1-3, X2-4, A-3, R-3), (X1-3, X2-4, A-3, R-4),
      (X1-3, X2-4, A-4, R=1), (X1-3, X2-4, A-4, R-2), (X1-3, X2-4, A-4, R-3),
      (X1-3, X2-4, A-4, R-4),
      (X1-4, X2-1, A-1, R-1), (X1-4, X2-1, A-1, R-2), (X1-4, X2-1, A-1,
      R-3), (X1-4, X2-1, A-1, R-4), (X1-4, X2-1, A-2, R-1), (X1-4, X2-1, A-2, R-2), (X1-4, X2-1, A-2, R-3), (X1-4, X2-1, A-2, R-4), (X1-4, X2-1, A-3, R-1),
      (X1-4, X2-1, A-3, R-2), (X1-4, X2-1, A-3, R-3), (X1-4, X2-1, A-3, R-4),
      (X1-4, X2-1, A-4, R-1), (X1-4, X2-1, A-4, R-2), (X1-4, X2-1, A-4, R-3),
      (X1-4, X2-1, A-4, R-4), (X1-4, X2-2, A-1, R-1), (X1-4, X2-2, A-1, R-2),
      (X1-4, X2-2, A-1, R-3), (X1-4, X2-2, A-1, R-4), (X1-4, X2-2, A-2, R-1),
      (X1-4, X2-2, A-2, R-2), (X1-4, X2-2, A-2, R-3), (X1-4, X2-2, A-2, R-4),
      (X1-4, X2-2, A-3, R-1), (X1-4, X2-2, A-3, R-2), (X1-4, X2-2, A-3, R-3),
      (X1-4, X2-2, A-3, R-4), (X1-4, X2-2, A-4, R-1), (X1-4, X2-2, A-4, R-2),
      (X1-4, X2-2, A-4, R-3), (X1-4, X2-2, A-4, R-4), (X1-4, X2-3, A-1, R-1),
      (X1-4, X2-3, A-1, R-2), (X1-4, X2-3, A-1, R-3), (X1-4, X2-3, A-1, R-4),
      (X1-4, X2-3, A-2, R-1), (X1-4, X2-3, A-2, R-2), (X1-4, X2-3, A-2, R-3),
      (X1-4, X2-3, A-2, R-4), (X1-4, X2-3, A-3, R-1), (X1-4, X2-3, A-3, R-2),
      (X1-4, X2-3, A-3, R-3), (X1-4, X2-3, A-3, R-4), (X1-4, X2-3, A-4, R-1),
      (X1-4, X2-3, A-4, R-2), (X1-4, X2-3, A-4, R-3), (X1-4, X2-3, A-4, R-4),
      (X1-4, X2-4, A-1, R-1), (X1-4, X2-4, A-1, R-2), (X1-4, X2-4, A-1, R-3),
      (X1-4, X2-4, A-1, R-4), (X1-4, X2-4, A-2, R-1), (X1-4, X2-4, A-2, R-2),
      (X1-4, X2-4, A-2, R-3), (X1-4, X2-4, A-2, R-4), (X1-4, X2-4, A-3, R-1),
      (X1-4, X2-4, A-3, R-2), (X1-4, X2-4, A-3, R-3), (X1-4, X2-4, A-3, R-4),
      (X1-4, X2-4, A-4, R-1), (X1-4, X2-4, A-4, R-2), (X1-4, X2-4, A-4, R-3),
      (X1-4, X2-4, A-4, R-4)
      (X1-6, X2-5, A-5, X-6), (X1-6, X2-5, A-5, X-7), (X1-6, X2-5, A-5,
      X-8), (X1-6, X2-5, A-5, X-9), (X1-6, X2-5, A-6, X-6), (X1-6, X2-5, A-6, X-7), (X1-6, X2-5, A-6, X-8), (X1-6, X2-5, A-6, X-9), (X1-6, X2-5, A-7, X-6),
      (X1-6, X2-5, A-7, X-7), (X1-6, X2-5, A-7, X-8), (X1-6, X2-5, A-7, X-9),
      (X1-6, X2-6, A-5, X-6), (X1-6, X2-6, A-5, X-7), (X1-6, X2-6, A-5, X-8),
      (X1-6, X2-6, A-5, X-9), (X1-6, X2-6, A-6, X-6), (X1-6, X2-6, A-6, X-7),
      (X1-6, X2-6, A-6, X-8), (X1-6, X2-6, A-6, X-9), (X1-6, X2-6, A-7, X-6),
      (X1-6, X2-6, A-7, X-7), (X1-6, X2-6, A-7, X-8), (X1-6, X2-6, A-7, X-9),
      (X1-6, X2-7, A-5, X-6), (X1-6, X2-7, A-5, X-7), (X1-6, X2-7, A-5, X-8),
      (X1-6, X2-7, A-5, X-9), (X1-6, X2-7, A-6, X-6), (X1-6, X2-7, A-6, X-7),
      (X1-6, X2-7, A-6, X-8), (X1-6, X2-7, A-6, X-9), (X1-6, X2-7, A-7, X-6),
      (X1-6, X2-7, A-7, X-7), (X1-6, X2-7, A-7, X-8), (X1-6, X2-7, A-7, X-9),
      (X1-6, X2-8, A-5, X-6), (Xl-6, X2-8, A-5, X-7), (X1-6, X2-8, A-5, X-8),
      (X1-6, X2-8, A-5, X-9), (X1-6, X2-8, A-6, X-6), (X1-6, X2-8, A-6, X-7),
      (X1-6, X2-8, A-6, X-8), (X1-6, X2-8, A-6, X-9), (X1-6, X2-8, A-7, X-6),
      (X1-6, X2-8, A-7, X-7), (X1-6, X2-8, A-7, X-8), (X1-6, X2-8, A-7, X-9),
      (X1-6, X2-9, A-5, X-6), (X1-6, X2-9, A-5, X-7), (X1-6, X2-9, A-5, X-8),
      (X1-6, X2-9, A-5, X-9), (X1-6, X2-9, A-6, X-6), (X1-6, X2-9, A-6, X-7),
      (X1-6, X2-9, A-6, X-8), (X1-6, X2-9, A-6, X-9), (X1-6, X2-9, A-7, X-6),
      (X1-6, X2-9, A-7, X-7), (X1-6, X2-9, A-7, X-8), (X1-6, X2-9, A-7, X-9),
      (X1-7, X2-5, A-5, X-6), (X1-7, X2-5, A-5, X-7), (X1-7, X2-5, A-5,
      X-8), (X1-7, X2-5, A-5, X-9), (X1-7, X2-5, A-6, X-6), (X1-7, X2-5, A-6, X-7), (X1-7, X2-5, A-6, X-8), (X1-7, X2-5, A-6, X-9), (X1-7, X2-5, A-7, X-6),
      (X1-7, X2-5, A-7, X-7), (X1-7, X2-5, A-7, X-8), (X1-7, X2-5, A-7, X-9),
      (X1-7, X2-6, A-5, X-6), (X1-7, X2-6, A-5, X-7), (X1-7, X2-6, A-5, X-8),
      (X1-7, X2-6, A-5, X-9), (X1-7, X2-6, A-6, X-6), (X1-7, X2-6, A-6, X-7),
      (X1-7, X2-6, A-6, X-8), (X1-7, X2-6, A-6, X-9), (X1-7, X2-6, A-7, X-6),
      (X1-7, X2-6, A-7, X-7), (X1-7, X2-6, A-7, X-8), (X1-7, X2-6, A-7, X-9),
      (X1-7, X2-7, A-5, X-6), (X1-7, X2-7, A-5, X-7), (X1-7, X2-7, A-5, X-8),
      (X1-7, X2-7, A-5, X-9), (X1-7, X2-7, A-6, X-6), (X1-7, X2-7, A-6, X-7),
      (X1-7, X2-7, A-6, X-8), (X1-7, X2-7, A-6, X-9), (X1-7, X2-7, A-7, X-6),
      (X1-7, X2-7, A-7, X-7), (X1-7, X2-7, A-7, X-8), (X1-7, X2-7, A-7, X-9),
      (X1-7, X2-8, A-5, X-6), (X1-7, X2-8, A-5, X-7), (X1-7, X2-8, A-5, X-8),
      (X1-7, X2-8, A-5, X-9), (X1-7, X2-8, A-6, X-6), (X1-7, X2-8, A-6, X-7),
      (X1-7, X2-8, A-6, X-8), (X1-7, X2-8, A-6, X-9), (X1-7, X2-8, A-7, X-6),
      (X1-7, X2-8, A-7, X-7), (X1-7, X2-8, A-7, X-8), (X1-7, X2-8, A-7, X-9),
      (X1-7, X2-9, A-5, X-6), (X1-7, X2-9, A-5, X-7), (X1-7, X2-9, A-5, X-8),
      (X1-7, X2-9, A-5, X-9), (X1-7, X2-9, A-6, X-6), (X1-7, X2-9, A-6, X-7),
      (X1-7, X2-9, A-6, X-8), (X1-7, X2-9, A-6, X-9), (X1-7, X2-9, A-7, X-6),
      (X1-7, X2-9, A-7, X-7), (X1-7, X2-9, A-7, X-8), (X1-7, X2-9, A-7, X-9),
      (X1-8, X2-5, A-5, X-6), (X1-8, X2-5, A-5, X-7), (X1-8, X2-5, A-5,
      X-8), (X1-8, X2-5, A-5, X-9), (X1-8, X2-5, A-6, X-6), (X1-8, X2-5, A-6, X-7), (X1-8, X2-5, A-6, X-8), (X1-8, X2-5, A-6, X-9), (X1-8, X2-5, A-7, X-6),
      (X1-8, X2-5, A-7, X-7), (X1-8, X2-5, A-7, X-8), (X1-8, X2-5, A-7, X-9),
      (X1-8, X2-6, A-5, X-6), (X1-8, X2-6, A-5, X-7), (X1-8, X2-6, A-5, X-8),
      (X1-8, X2-6, A-5, X-9), (X1-8, X2-6, A-6, X-6), (X1-8, X2-6, A-6, X-7),
      (X1-8, X2-6, A-6, X-8), (X1-8, X2-6, A-6, X-9), (X1-8, X2-6, A-7, X-6),
      (X1-8, X2-6, A-7, X-7), (X1-8, X2-6, A-7, X-8), (X1-8, X2-6, A-7, X-9),
      (X1-8, X2-7, A-5, X-6), (X1-8, X2-7, A-5, X-7), (X1-8, X2-7, A-5, X-8),
      (X1-8, X2-7, A-5, X-9), (X1-8, X2-7, A-6, X-6), (X1-8, X2-7, A-6, X-7),
      (X1-8, X2-7, A-6, X-8), (X1-8, X2-7, A-6, X-9) (X1-8, X2-7, A-7, X-6),
      (X1-8, X2-7, A-7, X-7), (X1-8, X2-7, A-7, X-8), (X1-8, X2-7, A-7, X-9),
      (X1-8, X2-8, A-5, X-6), (X1-8, X2-8, A-5, X-7), (X1-8, X2-8, A-5, X-8),
      (X1-8, X2-8, A-5, X-9), (X1-8, X2-8, A-6, X-6), (X1-8, X2-8, A-6, X-7),
      (X1-8, X2-8, A-6, X-8), (X1-8, X2-8, A-6, X-9), (X1-8, X2-8, A-7, X-6),
      (X1-8, X2-8, A-7, X-7), (X1-8, X2-8, A-7, X-8), (X1-8, X2-8, A-7, X-9),
      (X1-8, X2-9, A-5, X-6), (X1-8, X2-9, A-5, X-7), (X1-8, X2-9, A-5, X-8),
      (X1-8, X2-9, A-5, X-9), (X1-8, X2-9, A-6, X-6), (X1-8, X2-9, A-6, X-7),
      (X1-8, X2-9, A-6, X-8), (X1-8, X2-9, A-6, X-9), (X1-8, X2-9, A-7, X-6),
      (X1-8, X2-9, A-7, X-7), (X1-8, X2-9, A-7, X-8), (X1-8, X2-9, A-7, X-9),
      (X1-9, X2-5, A-5, X-6), (X1-9, X2-5, A-5, X-7), (X1-9, X2-5, A-5,
      X-8), (X1-9, X2-5, A-5, X-9), (X1-9, X2-5, A-6, X-6), (X1-9, X2-5, A-6, X-7), (X1-9, X2-5, A-6, X-8), (X1-9, X2-5, A-6, X-9), (X1-9, X2-5, A-7, X-6),
      (X1-9, X2-5, A-7, X-7), (X1-9, X2-5, A-7, X-8), (X1-9, X2-5, A-7, X-9),
      (X1-9, X2-6, A-5, X-6), (X1-9, X2-6, A-5, X-7), (X1-9, X2-6, A-5, X-8),
      (X1-9, X2-6, A-5, X-9), (X1-9, X2-6, A-6, X-6), (X1-9, X2-6, A-6, X-7),
      (X1-9, X2-6, A-6, X-8), (X1-9, X2-6, A-6, X-9), (X1-9, X2-6, A-7, X-6),
      (X1-9, X2-6, A-7, X-7), (X1-9, X2-6, A-7, X-8), (X1-9, X2-6, A-7, X-9),
      (X1-9, X2-7, A-5, X-6), (X1-9, X2-7, A-5, X-7), (X1-9, X2-7, A-5, X-8),
      (X1-9, X2-7, A-5, X-9), (X1-9, X2-7, A-6, X-6), (X1-9, X2-7, A-6, X-7),
      (X1-9, X2-7, A-6, X-8), (X1-9, X2-7, A-6, X-9), (X1-9, X2-7, A-7, X-6),
      (X1-9, X2-7, A-7, X-7), (X1-9, X2-7, A-7, X-8), (X1-9, X2-7, A-7, X-9),
      (X1-9, X2-8, A-5, X-6), (X1-9, X2-8, A-5, X-7), (X1-9, X2-8, A-5, X-8),
      (X1-9, X2-8, A-5, X-9), (X1-9, X2-8, A-6, X-6), (X1-9, X2-8, A-6, X-7),
      (X1-9, X2-8, A-6, X-8), (X1-9, X2-8, A-6, X-9), (X1-9, X2-8, A-7, X-6),
      (X1-9, X2-8, A-7, X-7), (X1-9, X2-8, A-7, X-8), (X1-9, X2-8, A-7, X-9),
      (X1-9, X2-9, A-5, X-6), (X1-9, X2-9, A-5, X-7), (X1-9, X2-9, A-5, X-8),
      (X1-9, X2-9, A-5, X-9), (X1-9, X2-9, A-6, X-6), (X1-9, X2-9, A-6, X-7),
      (X1-9, X2-9, A-6, X-8), (X1-9, X2-9, A-6, X-9), (X1-9, X2-9, A-7, X-6),
      (X1-9, X2-9, A-7, X-7), (X1-9, X2-9, A-7, X-8), (X1-9, X2-9, A-7, X-9);
      and a prodrug thereof, a pharmaceutically acceptable salt or solvate of them.

### BEST MODE FOR CARRYING OUT THE INVENTION

The method of preparation of compound (I) are described below.

Compounds (I) may be readily synthesized by direct coupling of aryls in the presence of a palladium catalyst. For example, a compound of formula (II) (hereinafter referred to compound (II)) and a compound of formula (III) (hereinafter referred to compound (III)) may be directly connected in the presence of palladium catalyst to give a compound (I) (hereinafter referred to compound (I)). The conditions may be modified depending on the selection of Z (or L). In case of Suzuki reaction (Chemical Communication 1979, 866, Journal of Synthetic Organic Chemistry, Japan, 1993, Vol. 51, No. 11, pp91-100), boron is used as Z (or L). Modifications of Suzuki reaction wherein an alkylsilan, a halogenated zinc, or an alkylated tin is used instead of boron may be conducted in a similar manner (Still reaction: Chem. Int. Ed., 1986, 25, 508). Appropriate metal reagents may be selected depending on structures of intended compounds and starting materials. Such modified procedures are basically conducted in a similar manner to Suzuki reaction, and Suzuki reaction is exemplified to illustrate specific preparation of compounds (I). 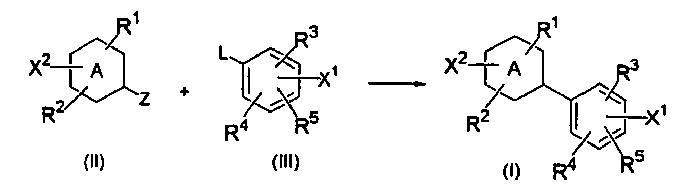 wherein one of L and Z is dihydroxyboryl (B(OH)₂), a di-(lower alkyl)boryl (BR₂), or a di-(lower alkoxy)boryl (B(OR)₂) wherein R is a lower alkyl, the other is a halogen, or -OSO₂(CqF_{2q+1}) wherein q is an integer of 0 to 4, and the remaining symbols are as defined above.

Compound (II) and compound (III) are reacted in the presence of a palladium catalyst (e.g. Pd(PPh₃)₄, PdCl₂(PPh₃)₂, PdC₂(OAc)₂ or PdCl₂(CH₃CN)₂, preferably Pd(PPh₃)₄) in a suitable solvent (e.g., benzene, toluene, N,N-dimethylformamide, dimethoxyethane, tetrahydrofuran, dioxane, ethanol and methanol, or a mixture of them and water) under a basic condition (K₃PO₄, NaHCO₃, NaOEt, Na₂CO₃, Et₄NCl, Ba(OH)₂, Cs₂CO₃, CsF, NaOH or Ag₂CO₃ is used as a base) at a temperature between room temperature and reflux temperature for a period of time from several ten minutes to several ten hours, to form compound (I) wherein the two rings are connected via a C-C bond.

Either one of the substituents, L or Z, is a boryl group such as dihydroxyboryl, a di-(lower alkyl)boryl, and a di-(lower alkoxy)boryl, and the other is a leaving group such as a halogen or -OSO₂(CqF_{2q+1}) wherein q is an integer of 0 to 4. Halogens and trifluoromethane-sulfonyloxy (hereinafter referred to as OTf) are favored, and bromine, iodine and OTf are most preferable.

Preferably, X¹, X², and R¹ to R⁵ on compounds (II) and (III) are groups that do not interfere with Suzuki reaction. Undesired products may be formed when an irrelevant leaving group such as halogen or sulfonate coexists. As far as the substituent L or Z is more reactive than other groups, the reaction proceeds normally.

When an interfering group exists in a molecule, the group may be protected with a suitable protecting group in advance if necessary. They are removed conventionally at a suitable following step. Specifically, the method described in Protective Group in Organic Synthesis 3rd. Ed. , John Wiley & Sons, NY (1999) may be used.

For example, when any of X¹, X², and R¹ to R⁵ is a hydroxy, then the group may be protected with methyl, methoxymethyl, methanesulfonyl, benzyl, tetrahydropyranyl, t-butyldimethylsilyl, or the like, and then removed at a suitable following step.

The above description illustrates the method of preparation of cyclic compound (I) from two discrete cyclic components (II) and (III). The process may be repeated in a similar manner using compound (I) and an additional aromatic cyclic compound, leading to various number of the aromatic rings at any arbitrary position. Further, when X¹ and X² are an aryl that may be optionally substituted or a heteroaryl that may be optionally substituted, then compounds (VI) and (V) may be reacted with compounds (IV) and (VII), respectively to obtain compound (I), as shown below. 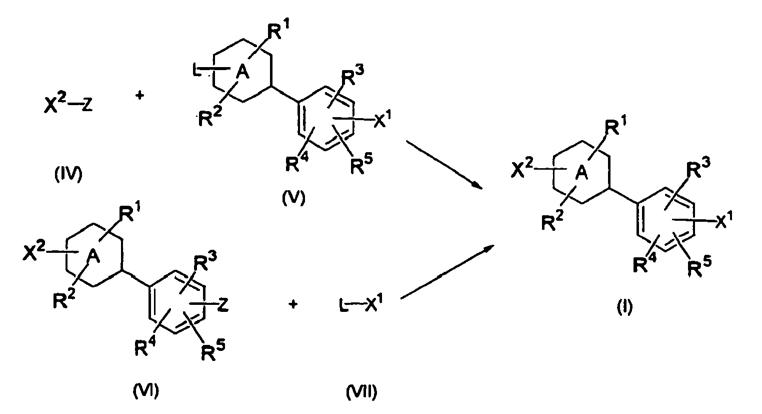 wherein the symbols are as defined above.

The process is an example to prepare intended compounds (I) by direct coupling of various cyclic compounds sequentially, which have been prepared. The process for preparing the nucleus can be used to prepare almost all of compounds (I), and is the most preferable one.

Alternative methods other than the above process may be used to prepare compounds (I). When, for example, X¹ in formula (I) is an aryl that may be optionally substituted or a heteroaryl that may be optionally substituted, and ring A is a 6-membered ring containing a nitrogen, then ring A may be constructed by conventional method of preparing a nitrogen-containing 6-membered ring to give compounds (I). Alternatively, when X¹ and X² comprise a heteroatom, then known reactions for preparing cyclic rings may be used to construct an intended cyclic ring compound.

Those methods are reviewed in many articles such as Comprehensive Heterocyclic Chemistry, O. M-Cohen, Ed. , Pergamon Oxford (1984) and Comprehensive Heterocyclic Chemistry II, A. R. Katri z ky, et al. , Ed. , Pergmon Oxford (1996).

It should be noted that the applicability of the processes based on the construction of heterocyclic ring would be less significant compared to the methods based on the coupling of rings since the number of the species of compounds (I) that can be prepared by the former process is significantly limited.

Contrary to the fact that many of conventional drugs help to increase in level of HDL-C relative to the decrease in level of LDL-C, pharmaceutical compositions of the invention enhance the expression of apoAI directly to effect increasing the level of HDL-C, which activate a reverse cholesterol transport activity of HDL, an anti-inflammatory activity and an anti-coagulant activity, or the like. As a result, the compositions of the invention are useful for preventing and/or treating dyslipidemia, arteriosclerotic diseases and various cardiovascular diseases associated with them, which are caused by decreased level of HDL in plasma. "Dyslipidemia" specifically include conditions of lowered level of serum HDL, hypercholesteremia and hypertriglyceridemia; "arteriosclerotic diseases" specifically include arteriosclerosis, myocardial infarction, and cardiac incompetence. "Various cardiovascular diseases associated with the above diseases" include hyperuricemia, coronary artery diseases, ischaemic heart diseases, corneal opacity, cerebrovascular disease, and hereditary HDL deficiencies (Tangier disease, fish-eye disease).

When a compound according to the invention is administered as the pharmaceutical compositions, such compositions may be administered either orally or parenterally. For oral routes, the compositions may be formulated conventionally into usual dosage forms such as tablets, tablets, granules, powders, capsules, pills, solutions, syrups, buccals, sublinguals, or the like before administration. For parenteral administration, the compositions may be conventionally formulated into usual dosage forms such as injections, e.g., intramuscular or intravenous injections, suppositories; transdermal patches, inhalation, or the like.

An effective amount of a compound according to the invention may be admixed with various suitable pharmaceutical additives such as excipient, binding agent, wetting agent, disintegrating agent, lubricant, diluent, or the like to give pharmaceutical compositions, if necessary. In the case of injections, the ingredients are sterilized together with a suitable carrier to formulate the composition.

More specifically, the excipients include lactose, sucrose, glucose, starch, calcium carbonate, crystalline cellulose, or the like; the binding agents include methyl cellulose, carboxymethylcellulose, hydroxypropylcellulose, gelatine, polyvinyl pyrrolidone, or the like; the disintegrating agents include carboxymethylcellulose, sodium carboxymethyl cellulose, starch, sodium alginate, algae powder, sodium lauryl sulfate, or the like; the lubricants include talc, magnesium stearate or Macrogol, or the like. Base materials of the suppository may be for example cacao butter, Macrogol, methylcellulose, or the like. Solutions, emulsions or suspensions for injection may comprise a solubilizing agent, a suspending agent, an emulsifying agent, a stabilizing agent, a preserving agent, an isotonic agent, or the like as usually used. Compositions for oral administration may comprise a flavoring agent, an aromatic agent, or the like.

Dose or therapeutically effective amount of the compounds according to the invention for enhancing the expression of apoAI is preferably determined considering age and body weight of patients, sort and severity of diseases to be treated, route of administration, or the like. In the case of oral administration to an adult, the dose range is usually 1 to 100 mg/kg/day, preferably 5 to 30 mg/kg/day. In the case of parenteral administration, the dose differs largely depending on the route of administration, but the dose range is usually 0.1 to 10 mg/kg/day, preferably 1 to 5 mg/kg/day. The dosage unit may be administered to a subject once or several times per day.

Following examples and experiment are presented for purpose of further illustration of the invention, and they are not intended to limit the scope of the invention in any respect.

### EXAMPLES

### Example 1

### 3-(4-Biphenyl)-6-methoxypyridazine (I-27)

To 3-chloro-6-methoxypyridazine (2.02g), 4-biphenyl borate (3.32g), and Pd(PPh₃)₄ (485mg), were added an aqueous solution of sodium carbonate(2M, 16.8ml) and dimethoxyethane (28ml), and the mixture was heated at reflux for 2 hours. After cooling, water was added, and the mixture was extracted with chloroform. The organic layer was dried over magnesium sulfate, and the solvent was evaporated. The residue was purified by silica gel (150 g), and recrystallized from ethyl acetate to give 2.98 g of the intended compound (81.3%).

### Example 2

### 2-[4-(3-Thienyl)phenyl]pyridine (I-36)

A solution of 2-bromopyridine (2.0 g) in anhydrous THF (50 ml) was cooled at -78°C in a nitrogen atmosphere, and a solution of tert-butyl lithium in pentane (1.64M, 15.0 ml) was added dropwise. The mixture was stirred for 10 minutes, and a 1M solution of zinc chloride in ether was dropwise added over 10 minutes. After the mixture was allowed to worm to room temperature over 2 hours, a solution of Pd(PPh₃)₄ (71 mg) and 1-bromo-4-iodobenzene (3.56 g) in anhydrous THF (20 ml) was added, and the mixture was stirred at room temperature for 3 days. To the reaction was added a 10% aqueous ammonia, and the mixture was extracted with ethyl acetate. The ethyl acetate solution was washed with saturated brine, and dried over magnesium sulfate, followed by evaporating the solvent. The residue was purified by silica gel chromatography (70 g, hexane-ethyl acetate = 4: 1) to give 2.37 g of 2-(4-bromophenyl]pyridine (79.9%).

To the aliquot of 2-(4-bromophenyl]pyridine (702 mg) were added 3-thiopheneboronic acid (460 mg), Pd(PPh₃)₄ (104 mg), sodium carbonate (2M, 3.6 ml), and DME (6ml), and the mixture was heated at reflux for 2 hours. After cooling, water was added, and the mixture was extracted with ethyl acetate. The organic layer was dried over magnesium sulfate. The solvent was evaporated, and the residue was purified by silica gel chromatography (40 g, hexane-ethyl acetate = 4: 1) to give crude materials, which were recrystallized from ethyl acetate giving 457 mg of the intended compound (64.3%).

In a similar manner, other compounds (I) were prepared, of which chemical structures and physiological constants are tabulated in the following tables

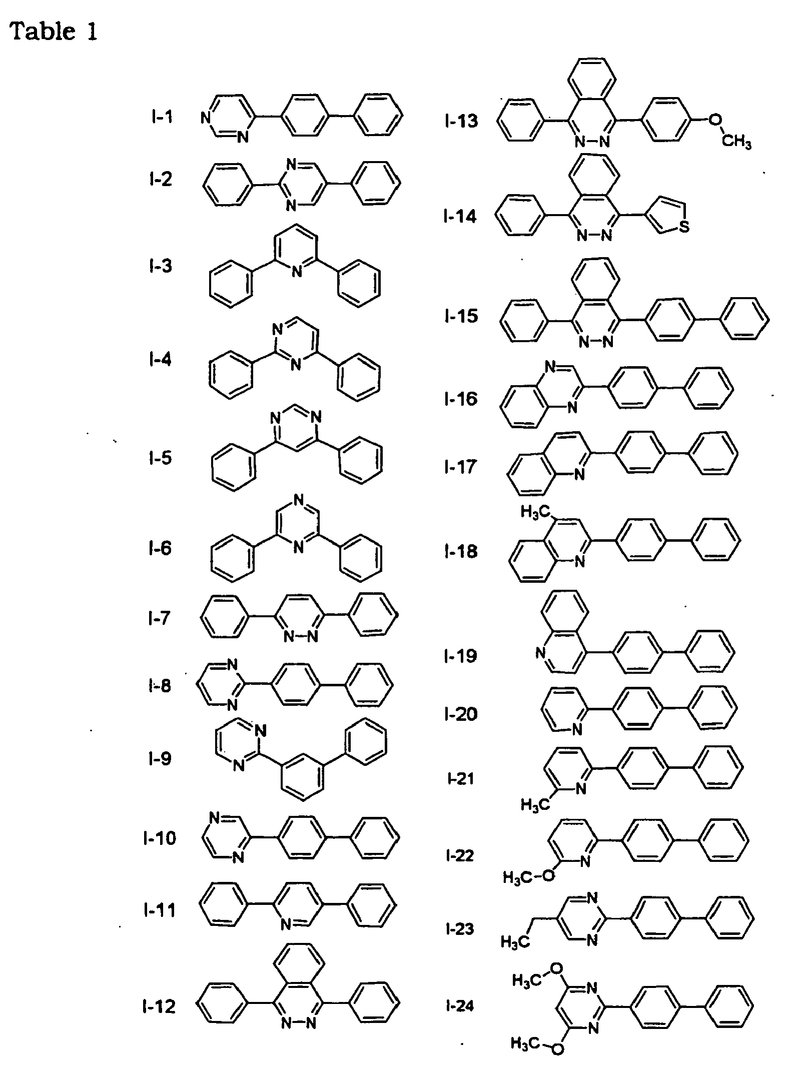

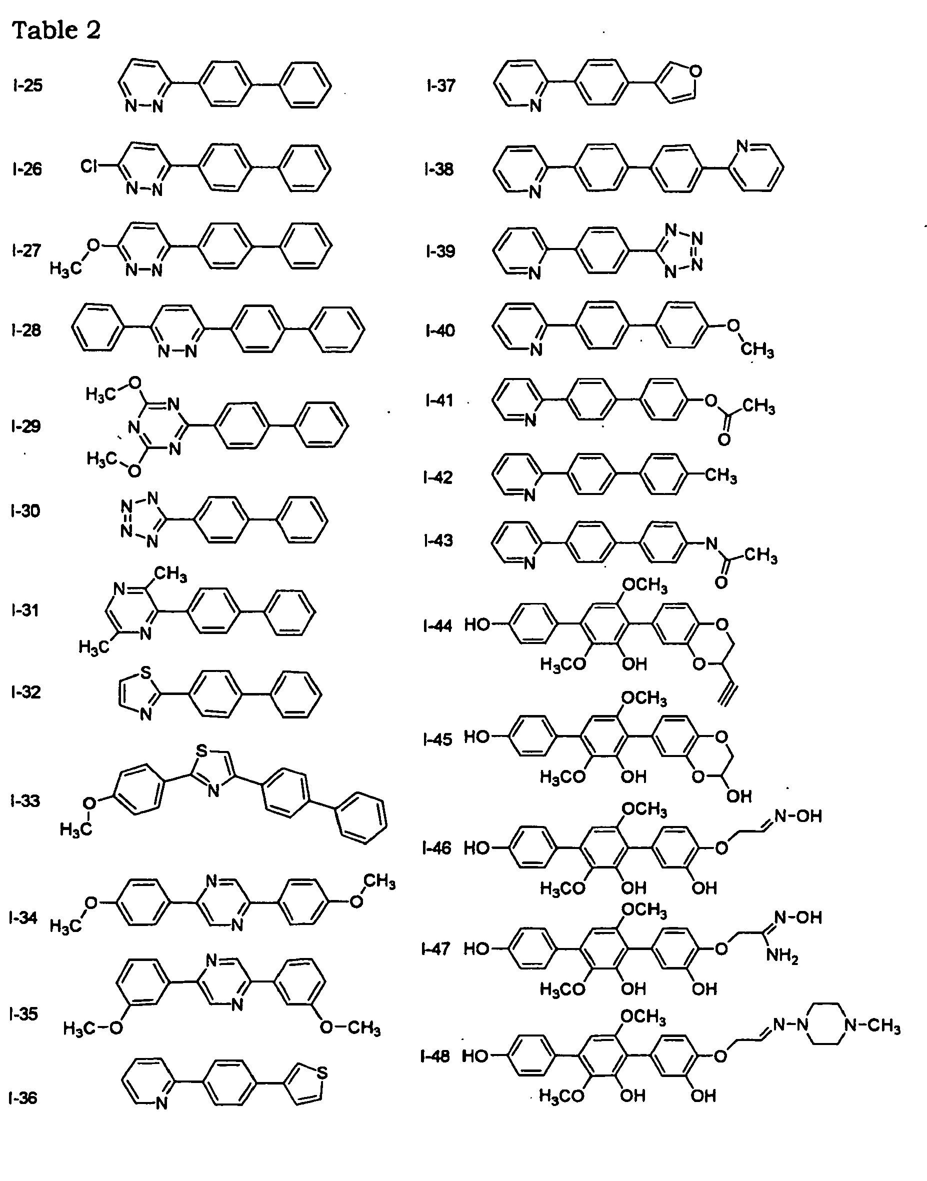

**Table 3**

| | m.p. (°C) | Molecular formula | Elemental analysis (Calculated) | Elemental analysis (Found) | NMR*2 |
|---|---|---|---|---|---|
| I-1 | Specs | | | | |
| I-2 | 185-186 | C16H12N2 | C, 82.73; H, 6.21; N, 12.06 | C, 82.60; H, 5.07; N, 12.08 | 7.43-7.67(8H, m), 8.46-8.63(2H, m), 9.03(2H, s) |
| I-3 | 75-77 | C17H13N | C, 88.28; H, 5.67; N, 6.06 | C, 88.28; H, 5.72; N, 6.26 | 7.39-7.65(6H, m), 7.67.7.73(2H, m), 7.78.7.86(1H, m) , 8.12-8.20(4H, m) |
| I-4 | 66-69 | C16H12N2 | C, 82.73; H, 5.21; N, 12.06 | C, 82.81; H, 5.20; N, 12.07 | 7.49.7.59(6H, m), 7.61(1H, d, J=5.2), 8.20-8.28(2H, m), 8.56-8.64(2H, m), 8.84(1H, d, J=5.2) |
| I-5 | 91-92 | C16H12N2 | C, 82.73; H, 5.21; N, 12.06 | C, 82.66; H, 5.21; N, 11.97 | 7.50-7.60(6H, m), 8.10.8.22(5H, m), 9.33(1H, s) |
| I-6 | 88.6-90 | C16H12N2 | C, 82.73; H, 5.21; N, 12.06 | C, 82.59; H, 5.28; N, 12.01 | 7.44-7.60(6H, m), 8.10.8.22(4H, m), 8.97(2H, s) |
| I-7 | 218-220 | C16H12N2 | C, 82.73; H, 5.21; N, 12.06 | C, 82.57; H, 5.18; N, 12.47 | 7.45-7.60(6H, m), 7.94(2H, s), 8.18-8.21(4H, m) |
| I-8 | 112-113 | C16H12N2 | C, 82.73; H, 5.21; N, 12.06 | C, 82.69; H, 5.19; N, 12.01 | 7.20(1H, t, J=4.8), 7.32-7.52(3H, m), 7.64-7.78(4H, m), 8.48-8.56(2H, m), 8.83(2H, d, J=4.8). |
| I-9 | 46-48 | C16H12N2 | C, 82.73; H, 5.21; N, 12.06 | C, 82.92; H, 5.24; N, 12.15 | 7.21(1H, d, J=4.8), 7.32-7.52(3H, m), 7.57(1H, t, J=8 .0), 7.67-7.77(3H, m), 8.44(1H, m), 8.72(1H, t. J=1.5 8.84(2H, d, J=4.8) |
| I-10 | 177-178 | C16H12N2 | C, 82.73; H, 6.21; N, 12.06 | C, 82.89; H, 5.23, 7.35-7.54(3H, N, 11.89 | m), 7.63-7.70(2H, m), 7.76(2H, d, J=8. 4), 8.11(2H, d, J=8.4), 8.52(1H, d, J=2.4), 8.66(1H, d d, J=1.8 and 2.4), 9.09(1H, d, J=1.8) |
| I-11 | 176-77 | C17H13N | C, 88.28; H, 5.67; N, 6.06 | C, 88.57; H, 5.66; N. 6.10 | 7.38-7.56(6H, m), 7.61-7.69(2H, m), 7.81(1H, d, J=8. 1), 7.96(1H, dd, J=2.4 and 8.1), 8.11-8.19(2H, m), 8. 94(1H, d, J=2.4) |
| I-12 | 203-205 | C20H14N2 0.6CHl3 | C, 69.90; H, 4.16; N, 7.91; Cl, 18.03 | C, 69.51; H, 3.99; N, 8.04; Cl, 14.26 | 8.16-8.13(2H, m), 7.87-7.80(6H, m), 7.62-7.57(6H, m) |
| I-13 | 175-7 | C21H16N2O FW 312.37 | C, 80.75; H, 6.16; N, 8.97 | C, 79.41; H, 5.14; N, 8.76 | 8.20-8.11(2H, m), 7.87-7.80(4H, m), 7.79(2H, d, J=8. 7), 7.61-7.56(3H, m), 7.12(2H, d, J=9.0), 3.92(3H, s) |
| I-14 | 186-8 | C18H12N2S 0.3CHCl3 | C, 67.80; H, 3.82; N, 8.64; S, 9.89 | C, 68.02; H, 3.82; 8.34(1H, N, 8.75; S, 10.02 | dd, J=1.8, 7.2), 8.14(1H, dd, J=2.1, 7.2), 7. 93-7.85(3H, m), 7.83-7.78(2H, m), 7.68(1H, dd, J=1.2 5.1), 7.61-7.54(4H, m) |

**Table 4**

| | | | | | |
|---|---|---|---|---|---|
| I-15 | 240-243 | C26H18N2-0. 6CHCl3 | C, 74.29; H, 4.36; N, 6.51 | C, 74.66; H, 4.18; N, 6.55 | 37-7.45(1H, m), 7.47-7.64(5H, m), 7.68-7.75(2H, m), 7.79-7.95(4H, m), 7.83(2H, d, J=8.4), 7.92(2H, d, J= 8.4), 8.12-8.27(2H, m) |
| I-16 | 126-127 | C20H14N2 | C, 85.08; H, 5.00; N, 9.92 | C, 85.33; H, 4.75; N, 9.87 | 7.38-7.52(1H, m), 7.46-7.52(2H,m), 7.67-7.70(2H, m), 7.73-7.78(2H, m), 7.81(2H, d, J=8.4), 8.12-8.19(2H, m), 8.30(2H, d, J=8.4), 9.38(1H, s) |
| I-17 | 180-182 | C21H15N | C, 89.65; H, 5.37; N, 4.98 | C, 89.87; H, 5.38; N, 6.06 | 7.35-7.40(1H, m), 7.47(2H, t. J=7.8), 7.53(1H, dt, J=1.2 and 7.5), 7.66-7.76(3H, m), 7.76(2H, d, J=8.1), 7.83 (1H, d, J=8.4), 7.93(1H, d, J=8.4), 8.18(1H, d, J= 8.4), 8.23(1H, d, J=7.2), 8.26(2H, d, J=8.4) |
| I-18 | 153-156 | C22H17N | C, 89.46; H, 5.80; N, 4.74 | C, 89.43; H, 5.80; N, 4.79 | 2.79(3H, s), 7.36-7.40(1H, m), 7.47(2H, t, J=7.6), 7.5 5(1H, dt, J=1.6 and 7.6), 7.68(2H, dd, J=2.1 and 7. 5), 7.72-7.77(2H, m), 7.75(2H, d, J=8.1), 8.01(1H, dd , J=1.5 and 8.4), 8.19(1H, dd, J=0.6 and 9.0), 8.24(2 H, d, J=8.4) |
| I-19 | 154-155 | C21H15N | C, 89.66; H, 5.37; N, 4.98 | C, 89.47, H, 6.29; N, 4.93 | 7.37-7.44(2H, m), 7.46-7.57(3H, m), 7.60(2H, d, J=8. 4), 7.66-7.80(3H, m), 7.70(2H, d, J=8.4), 8.01(1H, d, J=8.1), 8.20(1H, d, J=8.1), 8.97(1H, d, J=4.5) |
| I-20 | 144-145 | C17H13N | C, 88.28; H, 6.67; N, 6.06 | C, 87.99; H, 5.59; N, 6.24 | 7.20-7.28(1H, m), 7.41-7.52(3H, m), 7.61-7.82(4H, m) , 7.72(2H, d, J=8.4), 8.08(2H, d, J=8.4), 8.68-8.75(1 H, m) |
| I-21 | 119-120 | C18H15N | C, 88.13; H, 6.16; N, 5.71 | C, 88.35; H, 6.14; N, 5.68 | 2.64(3H, s), 7.10(1H, d, J=7.8), 7.32-7.50(3H, m), 7. 56(1H, d, J=7.8), 7.61-7.68(3H, m), 7.70(2H, d, J=9. 0), 8.07(2H, d, J=9.0) |
| I-22 | 134-136 | C18H15NO | C, 82.73; H, 5.79; N, 5.36 | C, 82.67; H, 5.77; N, 5.49 | 4.06(3H, s), 6.70(1H, d, J=8.4), 7.33-7.51(4H, m), 7. 60-7.68(3H, m), 7.69(2H, d, J=8.4), 8.13(2H, d, J=8. 4) |
| I-23 | 92-94 | C18H16N2 | C, 83.04; H, 6.19; N, 10.76 | C, 82.91; H, 6.21; N, 10.81 | 1.33(3H, t, J=7.5), 2.70(2H, q, J=7.5), 7.33-7.41(1H, m), 7.42-7.51(2H, m), 7.64-7.71(2H, m), 7.73(2H, d, J=8.7), 8.49(2H, d, J=8.7), 8.67(1H, s) |
| I-24 | 137-139 | C18H16N202 | C, 73.96; H, 5.52; N, 9.58 | C, 74.18; H, 5.70; N, 9.44 | 4.07(6H, s), 5.98(1H, s), 7.32-7.41(1H, m), 7.42-7.51( 2H, m), 7.62-7.69(2H, m), 7.70(2H, d, J=8.4), 8.53(2 H, d, J=8.4) |

**Table 5**

| | | | | | |
|---|---|---|---|---|---|
| I-25 | 190-191 | C16H12N2 | C, 82.73; H, 5.21; N, 12.06 | C, 82.60; H, 5.02; N, 12.28 | 7.35-7.43(1H, m), 7.44-7.52(2H, m), 7.56(1H, dd, J=4 .5 and 8.7), 7.63-7.70(2H, m), 7.77(2H, d, J=8.7), 7. 92(1H, dd, J=1.5 and 8.7), 8.18(2H, d, J=8.7), 9.18( 1H, d, J=4.5) |
| I-26 | 249-260 | C16H11N2Cl | C, 72.05; H, 4.16; N, 10.50; Cl, 13. 29 | C, 72.52; H, 4.18; N, 10.80; Cl, 12. 0), 86 | 7.36-7.43(1H, m), 7.44-7.53(2H, m), 7.69(1H, d, J=9. 7.62-7.70(2H, m), 7.77(2H, d, J=8.7), 7.88(1H, d, J=9.0), 8.14(2H, d, J=8.7) |
| I-27 | 222-223 | C17H14N20 | C, 77.84; H, 6.38; N, 10.68 | C, 77.88; H, 5.41; N, 10.68 | 4.21(3H, s), 7.07(1H, d, J=9.3), 7.34-7.42(1H, m), 7. 43-7.51(2H, m), 7.63-7.7.70(2H, m), 7.74(2H, d, J=8. 7), 7.83(1H, d, J=9.3), 8.10(2H, d, J=8.7) |
| I-28 | 260-261 | C22H16N2 | C, 85.69; H, 5.23; N, 9.08 | C, 85.77; H, 5.42; N, 9.15 | 7.35-7.43(1H, m), 7.44-7.61(5H, m), 7.62-7.73(2H, m) , 7.80(2H, d, J=8.7), 7.93-8.02(2H, m), 8.14-8.21(2H, m), 8.27(2H, d, J=8.7) |
| I-29 | 117-119 | C17H16N3O2 | C, 69.61; H, 5.15; N, 14.33 | C, 69.73; H, 5.03; N, 14.44 | 4.15(6H, s), 7.36-7.43(1H, m), 7.44-7.52(2H, m), 7.63 -7.70(2H, m), 7.72(2H, d, J=8.7), 8.57(2H, d, J=8.7) |
| I-30 | 249-250(d) | C13H10N4 | C, 70.26; H. 4.54; N, 26.21 | C, 70.27; H, 4.51; N, 25.17 | 8.12(2H, d, J=8.7), 7.76(2H, d, J=8.7), 7.65(2H, d, J =8.1), 7.51-7.46(2H, m), 7.43-7.40(1H, m), 3.21(1H, b |
| I-31 | 71-75 | C18H16N2 | C, 83.04; H, 6.19; N, 10.76 | C, 82.79; H, 6.01; N, 10.85 | 2.60(3H, s), 2.64(3H, s), 7.35-7.42(1H, m), 7.44-7.51( 2H, m), 7.62-7.68(2H, m), 7.65(2H, d, J=8.4), 7.71(2 H, d, J=8.4), 8.34(1H, s) |
| I-32 | 101-102 | C15H11NS | C, 75.92; H, 4.67; N, 5.90; S, 13.51 | C, 76.93; H, 4.67; N, 5.78; S, 13.45 | 7.35(1H, d, J=3.3), 7.36-7.42(1H, m), 7.43-7.51(2H, m), 7.61-7.68(2H, m), 7.69(2H, d, J=8.4), 7.89(1H, d, J=3.3), 8.05(2H, d, J=8.4) |
| I-33 | 200-201 | C22H17NOS | C, 76.94; H, 4.99; N, 4.08; S, 9.34 | C, 76.92; H. 4.88; N, 4.17; S, 9.25 | 3.88(3H, s), 6.98(2H, d, J=8.7), 7.32-7.40(1H, m), 7. 42-7.50(2H, m), 7.44(1H, s), 7.63-7.73(4H, m), 8.00(2 H, d, J=8.7), 8.06(2H, d, J=8.7) |
| I-34 | 224-227 | C18H16N202 | C, 73.96; H, 5.52; N, 9.58 | C, 74.28; H, 5.45; N, 9.40 | 2) 3.84(6H, s), 7.10(4H, d, J=9.0), 8.14(4H, d, J=9.0) 9.20(2H, s) |
| I-35 | 171-173 | C18H16N202 | C, 73.96; H, 5.52; N, 9.58 | C, 74.16; H, 5.51; N, 9.67 | 2) 3.87(6H, s), 7.05-7.13(2H, m), 7.48(2H, t, J=8.1), 7.72-7.82(4H, m), 9.34(2H, s) |
| I-36 | 173-175 | C15H11NS | C, 75.92; H, 4.67; N, 5.90; S, 13.51 | C. 75.93; H, 4.50; N, 5.95; S, 13.52. 4 | 7.19-7.25(1H, m), 7.38-7.49(2H, m), 7.54(1H, dd, J=1 and 2.6), 7.72(2H, d, J=8.4), 7.76(2H, dd, J=1.0 a nd 4.8), 8.05(2H, d, J=8.4), 8.71(1H, d, J=4.8) |

**Table 6**

| | | | | | |
|---|---|---|---|---|---|
| I-37 | 140-141 | C15H11NO | C, 81.43; H, 5.01; N, 6.33 | C, 81.55; H, 4.97; N, 6.41 6), | 6.73-6.78(1H, m), 7.18-7.25(1H, m), 7.50(1H, t, J=1. 7.60(2H, d, J=8.4), 7.71-7.83(3H, m), 8.02(2H, d, J=8.4), 8.70(1H, d, J=4.8) |
| I-38 | 274-275 | C22H16N2 | C, 85.69; H, 5.23; N, 9.08 | C, 85.51; H, 5.13; N, 9.16 | 7.20-7.30(2H, m), 7.72-7.84(4H, m), 7.79(4H, d, J=8. 4), 8.11(4H, d, J=8.4), 8.73(2H, d, J=4.8) |
| I-39 | 254-255 | C12H9N5 | C, 64.56; H, 4.06; N, 31.37 | C, 64.42; H, 4.01; N, 31.08 | 2) 7.38-7.48(1H, m), 7.89-8.00(1H, m), 8.09(1H, d, J =8.0), 8.17(2H, d, J=8.8), 8.34(2H, d, J=8.8), 8.73(1 H, d, J=4.8) |
| 1-40 | 178-179 | C18H15NO | C, 82.73; H, 5.79; N, 5.36 | C, 82.76; H, 5.67; N, 6.43 | 3.87(3H, s), 7.00(2H, d, J=8.6), 7.18-7.26(1H, m), 7. 60(2H, d, J=8.6), 7.67(2H, d, J=8.6), 7.73-7.83(2H, m), 8.06(2H, d, J=8.6), 8.71(1H, d, J=4.8) |
| I-41 | 222-224 | C19H15NO | C, 83.49; H, 5.53; N, 6. 12 | C, 83.56; H, 5.41; N, 6. 27 | 265(3H, s), 7.22-7.32(1H, m), 7.76(4H, d, J=8,4), 7.77-7.82(2H, m), 8.00(2H d, J=8.4), 8.12(2H, d, J=8.4), 8.73(1H, d, J=4.8) |
| I-42 | 137-138 | C18H15N | C, 88.13; H, 6.16; N, 5. 71 | C, 87.66; H, 6.13; N, 68 | 5.241(3H, s), 7.20-7.26(1H, m), 7.27(2H, d, J=8.7), 7.56(2H, d, J=8.4) , 7.70(2H, d, J=8.4), 7.74-7.80(2H, m), 8.07(2H, d, J=8.7), 8.69-8.74 1H, m) |
| I-43 | 251-252 | C19H16N2O | C, 79.14; H, 5.69;N, 9. 71 | C, 79.06; H, 5.53;N, 78 9(3H, s) | 9. 8.66(1H, d, J=4.8), 8.00(2H, d, J=8.4), 7.84-7.51(2H, m), 7.69(2H, d J=8.4), 7.64(2H, d, J=8.7), 7.61(2H, d, J=8.7), 7.29-7.25(1H, m), 2.8 |

| | | | | | |
|---|---|---|---|---|---|
| NMR solvent 1) CDCl₃+CD₃OD 2)NMR DMSO-d6 3)D₂O | | | | | |

### Experiment 1

### Activity to enhance the expression of human apoAI

The promoter region of the gene encoding human apoAI was isolated, and ligated upstream the structure gene of firefly luciferase to construct a reporter plasmid. The reporter plasmid and a marker plasmid conferring the neomycin resistance were co-infected to cell lines derived from human hepatoma, HepG2 cells, and the cell lines were incubated in a selection medium comprising DMEM medium containing 10 % fetal calf serum supplemented with G418 (Final concentration: 0.7 mg/mL, Gibco) to give established strains that stably expressed the reporter molecule. The strains were seeded to a 96-well culture plates at a density of 50,000 cells per well, and incubated for 48 hours at 37°C under an atmosphere of 5% carbon dioxide. Then, a solution of the compounds according to the invention in DMSO was added to the wells at final concentrations of 0 to 10 µg/mL. After further incubation for 24 hours, the cells were added with a luciferase assay reagent (Piccagene LT 7.5 registered trade mark, Toyo Ink, KK), and the luciferase activity was determined using a luminometer (MicroBeta^{™} TRILUX, 1 sec/well, Wallac). The concentration of the compounds, which intensified the luciferase activity twice compared to that of control (DMSO without any compound of the invention added) was set as the minimal effective dose (MED). The results are shown in Table 7.

**Table 7**

| Compounds | MED (µM) |
|---|---|
| I-1 | 0.63 |
| I-2 | 0.2 |
| I-6 | 3.11 |
| I-7 | 0.04 |
| I-8 | 0.09 |
| I-10 | 0.71 |
| I-11 | 0.21 |
| I-16 | 0.1 |
| I-17 | 0.33 |
| I-18 | 1.1 |
| I-20 | 0.23 |
| I-21 | 2.3 |
| I-22 | 0.84 |
| I-23 | 0.27 |
| I-24 | 2.2 |
| I-25 | 0.29 |
| I-26 | 0.27 |
| I-27 | 0.06 |
| I-28 | 2.5 |
| I-29 | 2.5 |
| I-35 | 0.29 |
| I-36 | 1.3 |
| I-37 | 4.4 |
| I-40 | 0.16 |
| I-41 | 0.1 |
| I-42 | 0.09 |
| I-43 | 0.3 |
| I-44 | 0.6 |
| I-45 | 2.4 |
| I-48 | 1.6 |

The result of Experiment 1 shows that the compounds according to the invention can promote the function of the gene encoding human apoAI, thereby enhancing the expression of apoAI.

**Formulation 1 Tablets**

| | |
|---|---|
| compound (I) | 15 mg |
| starch | 15 mg |
| lactose | 15 mg |
| crystalline cellulose | 19 mg |
| polyvinyl alcohol | 3 mg |
| distilled water | 30 mL |
| calcium stearate | 3 mg |

The ingredients other than calcium stearate were mixed uniformly, and the mixture was powdered, granulated, and dried to give granules having a suitable size. Then, the calcium stearate was added and the mixture was compressed to give a tablet formulation.

**Formulation 2 Capsules**

| | |
|---|---|
| compound (I) | 10 mg |
| magnesium stearate | 10 mg |
| lactose | 80 mg |

The ingredients were homogeneously mixed to give powders or fine particles, which were formed into a powder formulation. This was filled in capsules to give a capsule formulation.

**Formulation 3 Granules**

| | |
|---|---|
| compound (I) | 30 g |
| lactose | 265 g |
| magnesium stearate | 5 g |

The ingredients were mixed thoroughly, and the mixture was compressed, powdered, granulated and sieved to give a granule formulation.

### INDUSTRIAL APPLICABILITY

As is apparent from the experiment as described above, the compounds according to the invention have an activity for enhancing the expression of apoAI. Thus, the compounds according to the invention are useful as pharmaceutical compositions for preventing and/or treating dyslipidemia or arteriosclerotic diseases, a method and use therefor.

## Claims

1. A pharmaceutical composition for enhancing the expression of apoAI, which comprises a compound of formula (I): 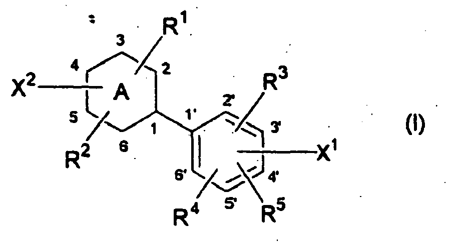 in which.
X¹ and X² are independently an aryl that may be optionally substituted, a heteroaryl that may be optionally substituted, a cycloalkyl that may be optionally substituted, an aryloxy that may be optionally substituted, a heteroaryloxy that may be optionally substituted, an arylthio that may be optionally substituted, a heteroarylthio that may be optionally substituted, a hydrogen, a halogen, a hydroxy, a lower alkyl that may be optionally substituted, a lower alkenyl that may be optionally substituted, a lower alkoxy that may be optionally substituted, a lower alkynyloxy that may be optionally substituted, a carboxy, a lower alkoxycarbonyl that may be optionally substituted, an acyl that may be optionally substituted, an amino that may be optionally substituted, or a carbamoyl that may be optionally substituted,
provided that at least one of X¹ and X² is an aryl that may be optionally substituted, or a heteroaryl that may be optionally substituted;
ring A is a pyridine ring, a pyrimidine ring, a pyrazine ring, a pyridazine ring, a triazine ring, a quinoline ring, a quinoxaline ring, or a phthalazine ring;
R¹, R², R³, R⁴ and R⁵ are independently a hydrogen, a halogen, a hydroxy, a lower alkyl that may be optionally substituted, a lower alkenyl that may be optionally substituted, a lower alkoxy that may be optionally substituted, a lower alkenyloxy that may be optionally substituted, a carboxy, a lower alkoxycarbonyl that may be optionally substituted, an acyl that may be optionally substituted, an amino that may be optionally substituted, or a carbamoyl that may be optionally substituted; a prodrug thereof, a pharmaceutically acceptable salt or solvate of them.

2. The pharmaceutical composition according to claim 1, in which X² is a phenyl that may be optionally substituted, or a heteroaryl that may be optionally substituted.

3. The pharmaceutical composition according to claim 1 or 2, in which X¹ is a hydrogen, a halogen, a hydroxy, a lower alkyl, a lower alkenyl, a lower alkoxy, or a phenyl.

4. The pharmaceutical composition according to any one of claims 1 to 3, which is used for prevention and/or treatment of dyslipidemia or arteriosclerotic diseases.

5. A method of enhancing the expression of apoAI, which comprises administrating a therapeutically effective amount of a compound of formula (I) as defined in claim 1, a prodrug thereof, a pharmaceutically acceptable salt or solvate of them to a patient expected to enhance the expression of apoAI.

6. Use of a compound of formula (I) as defined in claim 1, a prodrug thereof, a pharmaceutically acceptable salt or solvate of them for the manufacturing a medicament of enhancing the expression of apoAI.

7. Use of a compound of formula (I) as defined in claim 1, a prodrug thereof, a pharmaceutically acceptable salt or solvate of them for the manufacturing a medicament of treatment and/or prevention of dyslipidemia or arteriosclerotic diseases.
